# FASCICULE DE BREVET EUROPEEN

(11) **EP 0 424 519 B1**
(45) Date de publication et mention de la délivrance du brevet: **17.04.1996**
(21) Numéro de dépôt: 90908240.6
(22) Date de dépôt: 11.05.1990
(51) Int. Cl.: A61K 39/21

(54) **ANTIGENES DE LA GLYCOPROTEINE TRANSMEMBRANAIRE D'ENVELOPPE D'UN RETROVIRUS HUMAIN DU TYPE HIV-2 (ANTIGENES PRESENTANT AVEC EUX UNE PARENTE IMMUNOLOGIQUE)**
ANTIGENE EINES TRANSMEMBRANEN HÜLLENGLYCOPROTEINS EINES MENSCHLICHEN RETROVIRUS DES TYPS HIV-2 UND IMMUNOLOGISCH VERWANDTE ANTIGENE
ANTIGENES OF THE ENVELOPE TRANSMEMBRANE GLYCOPROTEIN OF A HUMAN RETROVIRUS OF THE HIV-2 TYPE, AND ANTIGENS PRESENTING WITH THEM AN IMMUNOLOGICAL RELATIONSHIP

(30) Priorité: 12.05.1989 FR 8906322; 25.05.1989 US 356459
(43) Date de publication de la demande: 02.05.1991
(73) Titulaire: INSTITUT PASTEUR, F-75724 Paris Cédex 15 (FR)
(72) Inventeur: HOVANESSIAN, Ara, F-93100 Montreuil (FR); REY, Maire-Anne, F-75004 Paris (FR); LAURENT, Anne, F-75007 Paris (FR); KRUST, Bernard, F-75012 Paris (FR); MONTAGNIER, Luc, F-92350 Plessis-Robinson (FR)
(74) Mandataire: Desaix, Anne
(86) Numéro de dépôt international: FR9000336
(87) Numéro de publication internationale: WO9013314

(56) Documents cités:
- EP-A- 269 520
- EP-A- 283 327
- EP-A- 314 534
- EP-A- 320 495
- CHEMICAL ABSTRACTS, vol. 111, 1989, Columbus, OH (US); M.A. REY et al., p. 364, no. 150196t/
- VIROLOGY, vol. 173, 1989; pp. 258-267/
- BIOLOGIE MOLECULAIRE DE LA CELLULE, Fammarion Médecine Science, Paris (FR), 1986; B. ALBERTS et al., pp. 264-265/

## Description

L'invention concerne des antigènes de la glycoprotéine transmembranaire d'enveloppe d'un rétrovirus humain du type HIV-2, des antigènes présentant avec eux une parenté immunologique et plus précisément des antigènes présents exclusivement lors de certaines phases de l'évolution in vivo d'une infection due à un rétrovirus humain HIV-2. Ces antigènes sont caractéristiques du processus de formation et de maturation de la glycoprotéine transmembranaire d'enveloppe de ce rétrovirus, et sont en conséquence impliqués dans le développement et la prolifération du rétrovirus HIV-2 et partant dans la propagation de l'infection.

L'invention a également trait à des anticorps reconnaissant ces antigènes, ainsi qu'à des compositions immunogènes contenant ces anticorps.

L'invention concerne en outre la préparation des antigènes précédents, ainsi que leur utilisation pour le diagnostic d'une infection due à un rétrovirus humain HIV-2.

Des agents étiologiques responsables du développement de syndrômes de lymphadénopathies (dont l'abréviation anglaise est SLA) ou concourant au développement de ces syndrômes ou responsables du développement de syndrômes d'immunodéficience acquise (SIDA) ont été isolés, identifiés et caractérisés.

On a désigné par HIV (abréviation anglaise de Human Immunodeficiency Virus) plusieurs rétrovirus humains susceptibles, dans certaines conditions, de provoquer un SLA éventuellement relayé par un SIDA chez l'homme.

Ainsi un premier virus dénommé LAV-1 ou HIV-1 a été isolé et décrit dans la demande de brevet EP.84/401.834 (publiée sous le n^{º} EP-A-0 138 669) du 14/09/84. Ce virus a également été décrit par F. Barre Sinoussi et al. (1).

Des variants de ce virus HIV-1 désignés par LAV ELI et LAV MAL, ont également été isolés, caractérisés et décrits dans la demande de brevet EP.84/401.834.

L'isolement et la caractérisation de rétrovirus appartenant à une classe distincte et n'ayant qu'une parenté immunologique réduite avec les précédents, ont été décrits dans la demande de brevet européen n° 87/400.151.4. publiée sous le n° 239.425. Ces rétrovirus qui ont été regroupés sous la désignation HIV-2, ont été isolés chez plusieurs malades africains présentant des symptômes d'une lymphadénopathie ou d'un SIDA.

L'étude de ces rétrovirus HIV l'isolement et l'analyse de leurs séquences nucléotidiques, ainsi que la caractérisation de leurs protéines antigéniques a permis d'effectuer des études de comparaison entre les différentes souches obtenues, au niveau de leur parenté ou au contraire de leur spécificité, tant structurales que fonctionnelles.

Ces rétrovirus HIV-1 et HIV-2 ont également été étudiés en comparaison avec un rétrovirus d'origine simienne (désigné par l'abréviation SIV ou STLV-III) et cette étude a permis de montrer une certaine parenté entre les protéines et glycoprotéines du rétrovirus HIV-2 et de ses variants et celles du rétrovirus SIV.
Une parenté est notamment remarquée au niveau de la protéine d'enveloppe de chacun de ces virus. On a en effet mis en évidence des réactions immunologiques croisées entre la glycoprotéine d'enveloppe du rétrovirus SIV et des anticorps dirigés contre la glycoprotéine d'enveloppe du rétrovirus HIV-2, alors que cette réaction de croisement immunologique ne se produit pas entre le rétrovirus SIV et le rétrovirus HIV-1 ni entre les rétrovirus HIV-1 et HIV-2.

Des résultats concernant la glycoprotéine d'enveloppe du rétrovirus HIV-2 codée par le gène env du génome de ce rétrovirus ont été donnés dans la demande de brevet européen n° 87/400.151.4. (publiée sous le n^{º} EP-A-0 239 425) précitée. Dans cette demande de brevet EP, cette glycoprotéine a été désignée par l'abréviation gp140 par référence à son poids moléculaire d'environ 140.000d. Il était précisé toutefois que le calcul du poids moléculaire était apprécié à ±10%. Dans une demande de brevet France précédente (n° 86/03881 publiée sous le n° 2.596.063) un précurseur de la gp140 désigné par gp160 (poids moléculaire de 160Kd ±10%) avait également été décrit.

Des investigations plus poussées relatives à la glycoprotéine d'enveloppe du rétrovirus HIV-2 ont été conduites et ont permis aux inventeurs de réaliser d'autres mesures des poids moléculaires donnés jusqu'à présent et en conséquence d'affiner les résultats, à partir des intervalles de poids moléculaires décrits ci-dessus. La glycoprotéine externe d'enveloppe présente, dans les particules virales de HIV-2 -dans les conditions opératoires de la présente inventionun PM d'environ 125000d (elle sera désignée par gp125). Dans ces mêmes conditions opératoires, le précurseur déjà mis en évidence et désigné par gp160 dans la demande de brevet FR.86/03881 a, selon les derniers résultats obtenus, un poids moléculaire d'environ 140000d. Il sera donc mentionné par "gp140" dans la présente demande de brevet.

Jusqu'à présent, l'existence de plusieurs phases de développement conduisant à l'obtention de glycoprotéines d'enveloppe sous une forme mature, ces glycoprotéines d'enveloppe matures comprenant une glycoprotéine externe d'enveloppe, gp125 et une glycoprotéine transmembranaire d'enveloppe gp36 a été mise en évidence différents stades dans le déroulement du cycle de réplication virale apparaissant lorsque des troubles pathogènes se développent à la suite d'une infection par un rétrovirus humain HIV-2 ont été identifiés à cet égard.

Des premiers résultats concernant l'existence et la caractérisation d'un précurseur de la glycoprotéine externe d'enveloppe gp300 de HIV-2 ont été obtenus. Cette glycoprotéine a été décrite dans une publication (13). Pour les besoins de la description, il sera fait référence dans la suite à cette glycoprotéine qui sera désignée par gp300. Les inventeurs ont maintenant mis en évidence et caractérisé une nouvelle glycoprotéine intervenant dans le cycle viral de HIV-2.

En parallèle, ils ont conduit des études similaires sur les cycles viraux des rétrovirus HIV-1 et SIV. Ces dernières études ont permis la mise en évidence de nouvelles propriétés très spécifiques communes aux rétrovirus HIV-2 et SIV et au contraire apparemment inexistentes lors du cycle de réplication virale de HIV-1.

L'invention concerne donc de nouveaux moyens pour détecter une infection due à un rétrovirus humain HIV-2 ou à un virus apparenté, ainsi que pour caractériser l'appartenance d'un rétrovirus au type des rétrovirus humains HIV-2.

Au vu des résultats obtenus, le cycle viral propre aux rétrovirus HIV-2 et SIV apparaît comme un processus complexe de réactions en chaîne dont l'ensemble seul pourrait conduire à la formation des glycoprotéines d'enveloppe du rétrovirus HIV-2 sous leur forme définitive. Lors de ce processus, de nouvelles protéines ou glycoprotéines antigéniques ont pu être reconnues, isolées et identifiées.

L'invention est donc relative à des glycoprotéines antigéniques produites à la suite d'une infection par un rétrovirus humain HIV-2, présentant une ossature peptidique en commun avec la glycoprotéine transmembranaire gp36 et susceptibles d'être dissociées lors du déroulement du cycle viral, en deux glycoprotéines transmembranaires gp36.

Une telle glycoprotéine, sous forme dimérisée correspondrait à la forme et à la conformation optimales et permettrait la fusion de la membrane virale avec la membrane cellulaire.

L'invention concerne donc une glycoprotéine caractérisée par les propriétés suivantes :
- elle est formée à partir d'un dimère d'une glycoprotéine transmembranaire d'enveloppe d'un rétrovirus HIV ou SIV,
- elle est capable de former un complexe immunologique du type antigène-anticorps, avec des anticorps dirigés contre une glycoprotéine antigénique gp80 ayant un poids moléculaire voisin de 80 kDa, reconnue par des anticorps polyclonaux diriges contre la glycoprotéine gp300 d'un rétrovirus humain HIV-2, et comprenant en association sous forme d'un dimère, deux entités de la glycoprotéine transmembranaire d'enveloppe gp36 codée par le génome d'un rétrovirus humain du type HIV-2, susceptible de provoquer un SLA ou un SIDA chez l'homme,
   - elle a un poids moléculaire du même ordre que celui de la susdite gp80 de HIV-2
   Selon une variante de réalisation, l'invention concerne une glycoprotéine antigénique gp80, caractérisée par les propriétés suivantes :
- elle a un poids moléculaire (PM) voisin de 80 kDa,
- elle est reconnue par des anticorps polyclonaux dirigés contre la glycoprotéine gp300 d'un rétrovirus humain HIV-2,
- elle comprend en association sous forme d'un dimère, deux entités de la glycoprotéine transmembranaire d'enveloppe gp36 codée par le génome d'un rétrovirus humain du type HIV-2, susceptible de provoquer un SLA ou un SIDA chez l'homme .

La glycoprotéine gp300 ci-dessus est la glycoprotéine décrite dans (13), elle a un poids moléculaire d'environ 300 KDa et est constituée par l'association de deux unités d'une glycoprotéine gp140 précurseur notamment de la glycoprotéine d'enveloppe gp125 codée par le gène env du génome d'un rétrovirus humain HIV-2 susceptible de provoquer un SLA ou un SIDA,
Après l'avoir isolée et caractérisée les inventeurs ont mis en évidence que le sérum de malades positifs pour les antigènes de HIV-2 reconnaissent la glycoprotéine gp80 dans des extraits cellulaires, ou dans des extraits viraux. La gp80 est un produit mature de la glycoprotéine transmembranaire, constituant une étape nécessaire pour la formation des particules virales infectieuses voire pathogènes. Les inventeurs ont donc déterminé qu'en bloquant le cycle viral normal de formation des particules du type HIV-2, notamment au niveau de la formation ou de la dissociation de la gp80, il est possible d'intervenir sur la propagation de l'infection. Un tel bloquage de l'infection pourrait être opéré en utilisant de la castanospermine, capable d'inhiber la maturation de la glycoprotéine gp80.

L'invention a donc pour objet une glycoprotéine caractérisée par les propriétés suivantes :
- elle est formée à partir d'un dimère d'une glycoprotéine transmembranaire d'enveloppe d'un rétrovirus HIV ou SIV
- elle a un poids moléculaire d'environ 80 kDa,
- elle est capable de former un complexe immunologique du type antigène-anticorps, avec des anticorps dirigés contre une glycoprotéine antigénique gp 80 ayant un poids moléculaire voisin de 80 kDa, reconnue par des anticorps polyclonaux dirigés contre la glycoprotéine gp300 d'un rétrovirus humain HIV-2, et comprenant en association sous forme d'un dimère, deux entités de la glycoprotéine transmembranaire d'enveloppe gp36 codée par le génome d'un rétrovirus humain du type HIV-2, susceptible de provoguer un SLA ou un SIDA chez l'homme.

Une glycoprotéine gp80 préférée selon l'invention est encore caractérisée par les propriétés suivantes :
- elle peut être associée à des cellules infectées par un rétrovirus humain du type HIV-2 ou/et à des virions ou/et à des virus du type HIV-2,
- elle n'est pas dissociée in vitro en présence de détergent non ionique tel que le Triton® X-100 1% et elle est dissociée in vitro pour former la glycoprotéine transmembranaire gp36 d'un rétrovirus du type HIV-2, en présence de détergent ionique tel que le SDS 1% à pH faiblement acide,
- elle peut être purifiée sur colonne d'immunoaffinité HIV-2 sérum Sépharose® et elle apparaît (lors d'une analyse par électrophorèse sur gel de polyacrylamide), 3 à 4 heures après le début du marquage des cellules infectées.

Entrent également dans le cadre de l'invention des protéines dérivées de la glycoprotéine gp80 définie ci-dessus et caractérisées en ce qu'elles correspondent à l'ossature peptidique non-glycosylée de cette glycoprotéine gp80.

Cette protéine, dépourvue des groupes de glycosylation de la gp80 est intéressante dans le cadre de l'invention, pour les propriétés qu'elle possède en commun avec la gp80.

Les études parallèles du cycle viral du rétrovirus simien SIV et du rétrovirus HIV-1 ont montré que le cycle de SIV comporte une étape de dimérisation sous forme d'une glycoprotéine gp65 d'un poids moléculaire d'environ 65 kDa, pouvant conduire à la formation de deux glycoprotéines transmembranaires d'enveloppe gp32.

En revanche, ce processus de dimérisation de la glycoprotéine transmembranaire d'enveloppe n'a pas été observé dans des échantillons biologiques infectés par HIV-1.

L'obtention d'une forme dimérisée, de la glycoprotéine transmembranaire d'enveloppe pourrait donc être associée à une infection spécifique par un rétrovirus du type HIV-2 ou un rétrovirus présentant une parenté immunologique avec ce dernier. Cette glycoprotéine constitue donc un moyen particulièrement adapté de détection d'une infection spécifique due au rétrovirus HIV-2 ou d'un rétrovirus apparenté.

L'invention a également trait à des anticorps polyclonaux ou monoclonaux reconnaissant spécifiquement d'une part, la gp300, la gp140, la gp125 et la gp80, présentes dans des cellules infectées par un rétrovirus du type HIV-2, d'autre part, la gp125, la gp80 et la gp36 présentes dans des extraits de rétrovirus du type HIV-2 et ne reconnaissant pas les protéines ou glycoprotéines de cellules saines ou de cellules infectées par un rétrovirus du type HIV-1.

Entrent également dans le cadre de l'invention des anticorps monoclonaux, caractérisés en ce qu'ils réagissent spécifiquement avec la glycoprotéine gp80 et qu'ils ne réagissent pas avec les protéines de cellules infectées par un rétrovirus du type HIV-1 ou avec des agrégats viraux de HIV-1.

Un autre anticorps monoclonal selon l'invention est caractérisé en ce qu'il reconnaît en outre les glycoprotéines gp300, gp140 et gp36 de HIV-2.

De façon particulièrement avantageuse, un troisième anticorps monoclonal conforme aux définitions précédentes est caractérisé en ce qu'il reconnaît un épitope commun à la glycoprotéine gp80, et à la séquence d'acides aminés suivante du peptide p39' :
VTAIEKYLQDQARLNSWGCAFRQVCH
L'invention vise aussi des anticorps dirigés contre la protéine dérivée de la gp80 et dépourvue des groupes glycosylés.

Les anticorps monoclonaux ci-dessus peuvent avoir des propriétés immunogènes et neutralisantes, et en agissant sur le processus de formation de la gp80, ils peuvent interrompre la prolifération du rétrovirus HIV.2.

Plusieurs méthodes pour la production des anticorps précédents peuvent convenir dans le cadre de la réalisation de l'invention. Par exemple, on aura recours à des cultures en ascite chez les animaux, notamment chez les rongeurs comme les murins. On pourra également produire les anticorps a partir de cultures cellulaires soit immobilisées ou encapsulées ou encore en suspension. On utilisera notamment la technique des hybridomes obtenus à partir de la fusion de lymphocytes d'un animal auquel on aura préalablement inoculé le rétrovirus HIV-2. Des lymphocytes de l'animal infecté sont alors prélevés et fusionnés avec des cellules myélomateuses choisies, pour former des hybrides, sélectionnés ensuite pour leur capacité à produire des anticorps spécifiques dirigés contre les protéines et glycoprotéines antigéniques décrites plus haut.

Un procédé de préparation préféré des anticorps monoclonaux de l'invention comprend les étapes suivantes :
- immunisation de souris type BALB-C si besoin à plusieurs reprises par injection intrapéritonéale avec la glycoprotéine gp80 purifiée,
- fusion de cellules de souris immunisées, notamment de cellules de rate avec des cellules myélomateuses, par exemple des cellules de type NS1, selon la méthode de Kohler et Milstein (10),
- sélection et récupération des hybridômes producteurs des anticorps monoclonaux recherchés.

Le criblage des hybridomes en vue de sélectionner ceux d'entre eux qui conduisent à la production des anticorps monoclonaux recherchés, peut être réalisé en utilisant des tests RIPA ou des analyses Western Blot.

Des anticorps monoclonaux spécifiquement adaptés à la réalisation de l'invention seront obtenus par l'une des méthodes ci-dessus décrites et leurs propriétés vis-à-vis de la gp80 ou d'une protéine analogue répondant aux définitions ci-dessus pourront être testées par comparaison avec les résultats décrits avec l'anticorps monoclonal 1H8 (Mab 1H8).

L'invention vise également des hybridomes producteurs des anticorps monoclonaux décrits précédemment.

Ces anticorps monoclonaux produits seront par exemple utilisés pour inactiver les protéines et/ou glycoprotéines avec lesquelles ils forment un complexe immunologique ou encore pour leur capacité à empêcher le déroulement normal de la formation des glycoprotéines finales de l'enveloppe de HIV-2.

Une autre application éventuelle de ces anticorps consisterait par exemple dans la détection d'antigènes viraux dans des préparations biologiques ou encore dans la purification de protéines et/ou glycoprotéines par exemple sur colonne d'affinité.

Ayant mis en évidence l'existence de la gp80 et après l'avoir isolée et caractérisée, les inventeurs ont mis au point des moyens pour effectuer le diagnostic d'une infection due à un rétrovirus humain du type HIV-2.

A cet effet l'invention concerne une composition antigénique pour le diagnostic spécifique in vitro d'une infection due à un rétrovirus humain du type HIV-2, dans un échantillon biologique susceptible de contenir des anticorps formés après ladite infection, caractérisée en ce qu'elle comprend au moins la glycoprotéine gp80 définie dans les pages précédentes.

L'échantillon biologique peut être un fluide biologique, notamment le sang ou le sérum ou encore un extrait tissulaire biologique. Les compositions ci-dessus sont utilisables pour la détection d'anticorps dans un échantillon biologique susdit.

L'invention vise donc également un procédé pour le diagnostic in vitro d'une infection due spécifiquement à un rétrovirus du type HIV-2 caractérisé par les étapes de :
- mise en contact d'un échantillon biologique susceptible de contenir des anticorps produits à la suite d'une infection par un rétrovirus du type HIV-2, avec une glycoprotéine gp80 répondant aux définitions ci-dessus ou avec une composition antigénique décrite ci-dessus, dans des conditions appropriées permettant la formation d'un complexe immunologique du type antigène-anticorps, et
- détection du complexe antigène-anticorps éventuellement formé.

L'invention concerne par ailleurs une composition immunogène caractérisée en ce qu'elle comprend au moins la glycoprotéine gp80 en association avec un véhicule pharmaceutique acceptable pour la constitution de vaccins.

Ces compositions peuvent être utilisées pour la fabrication de vaccins contre un rétrovirus du type HIV-2.

Une composition immunogène ainsi formée est dosée en protéines et/ou glycoprotéines antigéniques de façon à permettre l'administration d'une dose de 10 à 100µg par kilogramme.

L'invention se rapporte également à un procédé de préparation de la gp80 caractérisé par les étapes suivantes :
- lyse de cellules infectées par un rétrovirus humain HIV-2 et séparation du surnageant et des cellules infectées ou lyse des culots viraux préparés par centrifugation,
- incubation de l'extrait cellulaire et/ou de l'extrait viral sur une colonne d'immunoaffinité, sur laquelle est déposé un immunoadsorbant renfermant des anticorps purifiés, tels qu'obtenus à partir d'un sérum d'un malade infecté par le rétrovirus humain HIV-2, ce sérum ayant la capacité de réagir fortement avec les protéines d'enveloppe de HIV-2, lesdits anticorps étant fixés avantageusement sur un support adapté, en présence d'un tampon et pendant un temps suffisamment long pour obtenir la formation d'un complexe immunologique antigène-anticorps,
- lavage de la colonne avec un tampon pour éliminer les molécules non retenues sur le support,
- récupération des protéines antigéniques recherchées.

Selon un premier mode de réalisation de ce procédé de préparation, la récupération des glycoprotéines est faite par électrophorèse et par électroélution des protéines.

Les cellules infectées auront pu être obtenues in vitro à partir d'une culture de cellules infectées par HIV-2, en particulier en utilisant des lymphocytes T4 infectés par HIV-2. De telles cultures cellulaires peuvent être réalisées sur des lignées CEM.

Selon un autre mode de réalisation de ce procédé de préparation, la récupération des glycoprotéines est obtenue par:
- élution des protéines fixées sur la colonne d'immuno-affinité ci-dessus, et
- purification des produits ainsi élués sur une colonne de chromatographie contenant, fixés sur le support de séparation, des anticorps monoclonaux reconnaissant la gp80.

Dans un mode de réalisation particulier de l'invention la lyse des cellules infectées est réalisée dans une solution détergente, les anticorps compris dans l'immunoadsorbant sont fixés sur des billes d'agarose et on opère en présence d'un tampon d'accrochage.

Entre également dans le cadre de l'invention un nécessaire ou kit pour la détection d'anticorps dans le sérum ou dans un autre échantillon biologique d'un patient susceptible d'être infecté par un rétrovirus humain HIV-2, caractérisé en ce qu'il comprend:
- au moins une glycoprotéine antigénique gp80 ou une composition
- ou encore un mélange de ces différents constituants,
- des moyens permettant la réaction de formation du complexe immunologique entre les antigènes et les anticorps éventuellement présents dans l'échantillon biologique à tester, notamment si besoin un ou plusieurs tampons d'incubation,
- un échantillon de contrôle négatif,
- des moyens pour la révélation du complexe antigène-anticorps formé.

L'invention concerne également des glycoprotéines gp80 et gp65 marquées, de préférence avec des marqueurs radioactifs, enzymatiques, fluorescents, chimioluminescents ou des chromophores. L'invention fournit également une méthode pour la détection des cellules par le virus HIV-2. Cette méthode comprend le traitement d'un échantillon biologique comprenant des cellules susceptibles d'être infectées par HIV-2, de façon à exposer les protéines intracellulaires, et le test de détection des protéines intracellulaires exposées pour rechercher la présence de glycoprotéines gp80 de HIV-2. La détection des protéines intracellulaires exposées peut par exemple être faite par électrophorèse ou par un test immunologique avec des anticorps immunologiquement réactifs avec la gp80 de HIV-2. De tels anticorps ont par exemple été décrits dans les pages précédentes.

Selon un mode de réalisation particulier du diagnostic de la présence d'antigène gp80, l'échantillon biologique est un échantillon dépourvu des débris cellulaires.

L'invention fournit également les moyens pour détecter une infection spécifique par HIV-2 et pour la distinguer d'une infection due à HIV-1, à partir de dellules susceptibles de contenir des antigènes de HIV-2. Cette méthode comprend la mise en contact d'un échantillon biologique contenant des protéines intracellulaires obtenues à partir de cellules traitées d'un échantillon biologique que l'on veut tester, avec des anticorps dirigés contre la gp80. Une réaction éventuelle du type antigène-anticorps est ensuite recherchée pour détecter l'existence d'une infection par HIV-2.

D'autres caractéristiques et avantages de l'invention apparaîtront dans les exemples et les figures.

### LEGENDES DES FIGURES

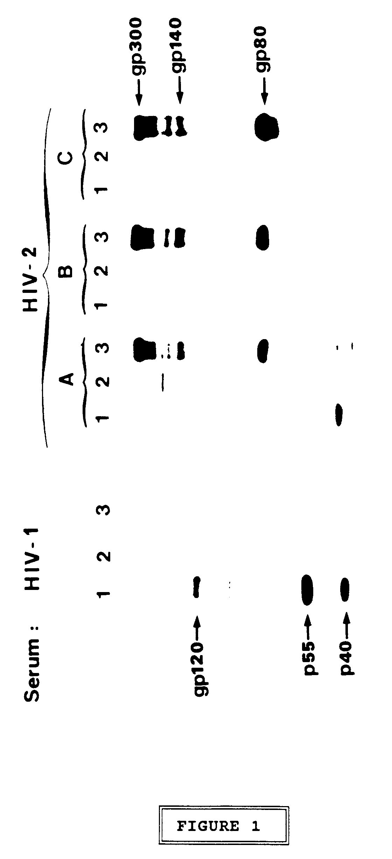

Figure 1:Identification d'une protéine spécifique de 80-kDa dans des cellules infectées par HIV-2 par analyse western blot en utilisant un sérum positif pour HIV-1 et 3 sérums positifs pour HIV-2 (A, B et C). Des extraits cellulaires de cellules CEM (correspondant aux produits de 10⁶ cellules) non infectées (lignes 2), de cellules infectées par HIV-1 (lignes 1) et de cellules infectées par HIV-2 (lignes 3) ont été analysés par électrophorèse sur gel de polyacrylamide 7,5% avant le test Western blot. Un autoradiographe est présenté sur la figure. Sur la gauche, les flèches indiquent la position de la glycoprotéine extracellulaire de HIV-1 (gp120) et des précurseurs de gag p55 et p40. Sur la droite on a noté la position des glycoprotéines spécifiques de HIV-2, gp300, gp140 et gp80.

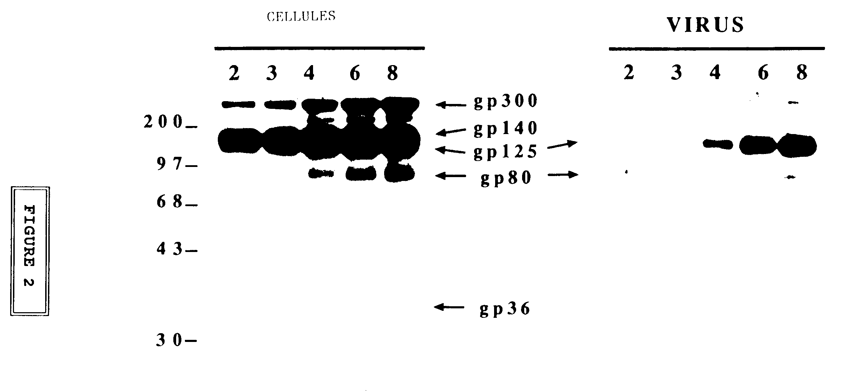

Figure 2:Synthèse des glycoprotéines liées à HIV-2. Les cellules infectées par HIV-2 ont été marquées avec [³H]glucosamine (200µCi/ml;4x10⁶ cellules/ml) pendant 2, 3, 4, 6 et 8 heures. A différents points, des extraits (produits de 10⁷ cellules) ont été préparés à partir de cellules infectées et à partir d'agrégats (synonyme de culots) viraux (préparés par centrifugation du milieu de culture à 100.000g). Des aliquotes de ces extraits (correspondant à 2 x 10⁶ cellules) ont été purifiées sur HIV-2 sérum-Sépharose® et les protéines marquées ont été analysées par électrophorèse sur gel de polyacrylamide (12,5%). Un fluorographe est présenté. Sur la gauche on a donné la position des marqueurs protéiques de poids moléculaire: la myosine: 200.000, la phosphorylase B: 97.000, la sérum albumine bovine: 68.000, l'ovalbumine: 43.000, et l'anhydrase carbonique: 30.000.

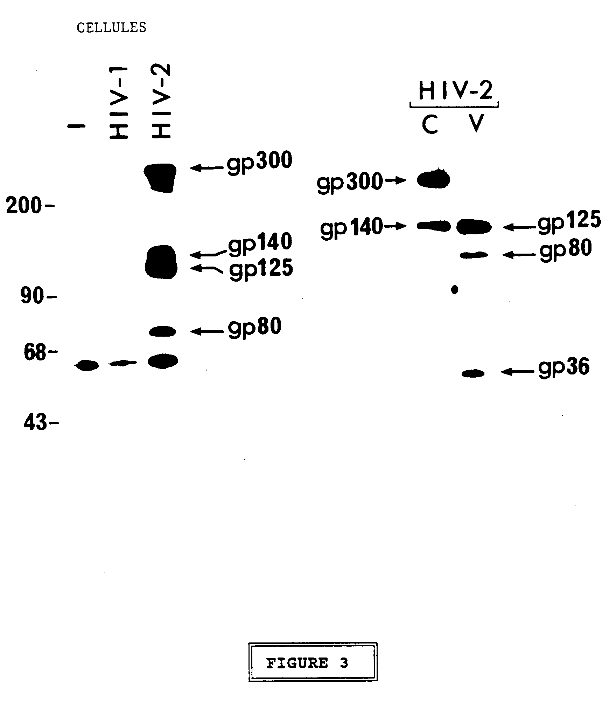

Figure 3:Analyse Western Blot avec des anticorps polyclonaux dirigés contre la gp300. Sur la gauche des extraits de cellules CEM non infectées (-) et infectées par HIV-1 ou HIV-2 sont présentés. Sur la droite des extraits de cellules CEM infectées par HIV-2: extraits cellulaires (ligne C) et agrégat viral (culot viral) (ligne V). Les échantillons ont été analysés par électrophorèse sur gel de polyacrylamide (gel à 7,5% sur la gauche ; gel à 12,5% sur la droite) avant l'analyse Western blot en utilisant des anticorps polyclonaux murins dirigés contre la protéine purifiée gp300 (anti-gp300). L'autoradiogramme est présenté, chaque échantillon correspond au produit de 10⁶ cellules.

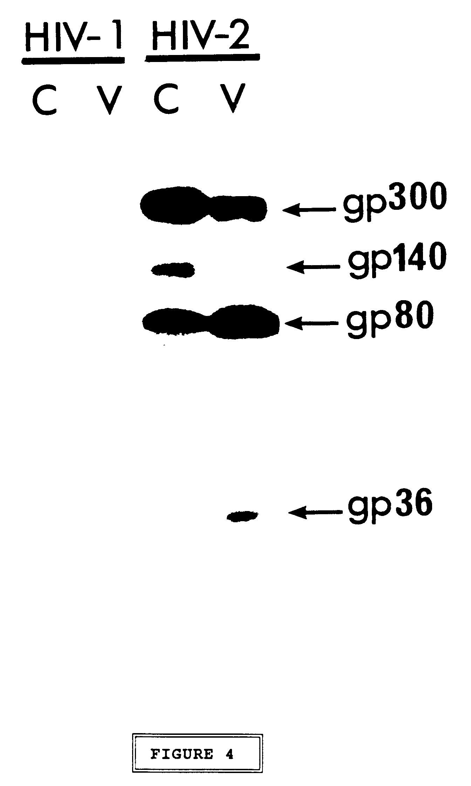

Figure 4:Une analyse Western blot en utilisant un anticorps monoclonal mAb 1H8 est présentée. Les extraits de cellules infectées par HIV-1 ou HIV-2 (lignes C) et les extraits d'agrégats viraux (lignes V) ont été analysés en électrophorèse sur gel de polyacrylamide 12,5% avant le test Western blot en utilisant l'anticorps mAb 1H8. L'autoradiographe est présenté, l'anticorps monoclonal était dirigé contre la glycoprotéine transmembranaire d'enveloppe de HIV-2_{ROD}.

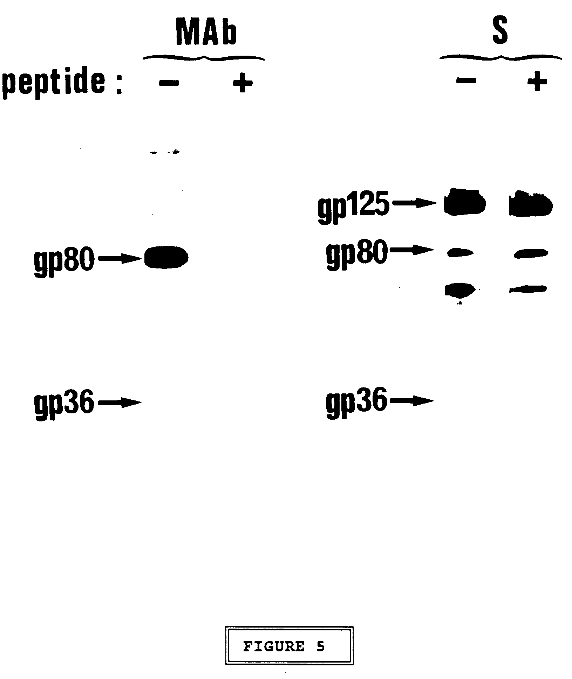

Figure 5:Le peptide p39' bloque la liaison entre l'anticorps mAb 1H8 et la gp80. Des extraits d'agrégats viraux de HIV-2 ont été analysés par Western blot en utilisant l'anticorps 1H8 (section mAb) ou des anticorps polyclonaux anti-gp300 (section S). L'incubation avec chaque anticorps a été réalisée en l'absence (lignes -) ou en présence (lignes +) de 1 µg/ml de peptide p39'. Les résultats de l'autoradiographie sont présentés.

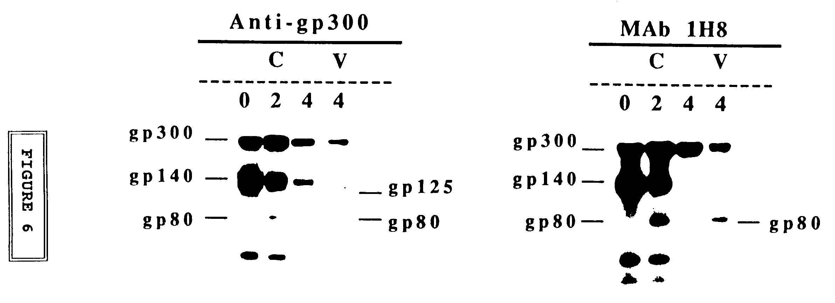

Figure 6:Des expériences de "Pulse chase" (marquage, suivi du marqueur) montrant la production de gp80 dans des cellules infectées par HIV-2 sont présentées. Les cellules CEM infectées par HIV-2 ont été marquées par la [³⁵S]méthionine (200 µCi/ml ; 4x10⁶ cellules/ml) pendant 30 minutes (lignes 0). Le marquage radioactif a été suivi dans le milieu de culture contenant 5mM de méthionine froide pendant 2 et 4 heures (lignes 2 et 4). Le milieu de culture a été centrifugé à 100.000g au bout de 4 heures et les agrégats ont été extraits. Tous les échantillons ont été immunoprécipités en utilisant des anticorps anti-gp300 ou des anticorps mAb 1H8. Les protéines marquées des préparations du complexe immun ont été éluées sur le tampon d'électrophorèse et analysées par électrophorèse sur gel de polyacrylamide 7,5%. Un fluorographe est présenté, dans lequel C et V correspondent aux extraits cellulaires et viraux respectivement. Chaque échantillon représente le produit de 10⁶ cellules.
Figure 7:
   a/ incorporation de glucosamine marquée et de fucose dans gp80. Les cellules CEM infectées par HIV-2 marquées avec [³H] glucosamine (200 µCi/ml) ou avec [³H] fucose (200 µCi/ml) ont été testées par immunoprécipitation en utilisant des anticorps polyclonaux anti-gp300 (lignes S) ou des anticorps monoclonaux mAb 1H8 (lignes M). Tous ces échantillons ont été analysés par électrophorèse sur gel de polyacrylamide 12,5%. Le fluorographe est présenté.
   b/ effet de la castanospermine sur la production de gp125 et gp80. Des cellules infectées par HIV-2 ont été marquées (16 heures) avec la [³⁵S] méthionine (200µCi/ml ; 4x10⁶ cellules/ml) en l'absence (lignes -) ou en présence (lignes +) de castanospermine (1mM). Les extraits du milieu de culture contenant les particules virales ont été purifiés sur colonne d'immunoaffinité en utilisant HIV-2 sérum-sépharose® et les protéines purifiées ont été testées par électrophorèse sur gel de polyacrylamide 12,5%. Le fluorographe correspondant est présenté.

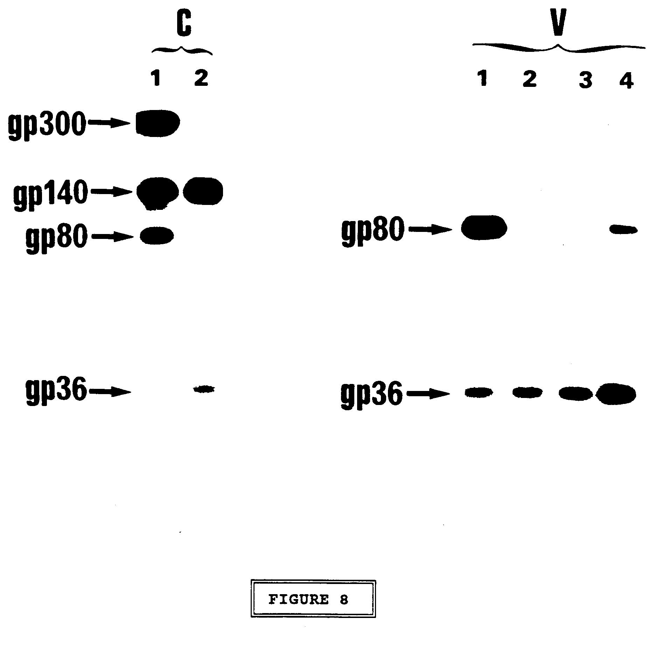

Figure 8:Dissociation de gp80. Section C: extraits de cellules CEM infectées par HIV-2 marquées à la [³⁵S] méthionine testés par immunoprécipitation en utilisant un anticorps monoclonal mAb 1H8 (ligne 1). Une autre aliquote de la même préparation d'extraits cellulaires a été d'abord chauffée (95°C, 5 minutes) en présence de 1% SDS. Ensuite elle a été diluée 10 fois dans un tampon RIPA avant le test d'immunoprécipiation (ligne 2). Les préparations de complexe immun ont été analysées par électrophorèse. Chaque échantillon correspond à l'extrait de 10⁶ cellules. Section V: des agrégats de virus HIV obtenus à partir de cellules marquées à la [³⁵S] méthionine (chacun correspondant au produit de 10⁷ cellules) ont été suspendus dans différents tampons: (1) tampon de lyse contenant Triton (10mM Tris-HCl pH 7,6, 150mM NaCl, 1mM EDTA, 1% (V/V) Triton® X-100 et 100 unités/ml d'aprotinine). (2) tampon de lyse contenant SDS (puis chauffé comme dans (1) mais contenant 1% (v/v) SDS à la place du Triton® X-100); (3) tampon de lyse contenant SDS puis chauffé à 95° pendant 5 minutes; (4) tampon RIPA (le même que dans (1) modifié aussi par 0,1% (v/v) SDS et 0,2% (v/v) déoxycholate). Tous ces échantillons ont ensuite été immunoprécipités en utilisant un anticorps mAb 1H8 et les protéines marquées ont été analysées par électrophorèse sur gel de polyacrylamide 12,5%. Le fluorographe est présenté.

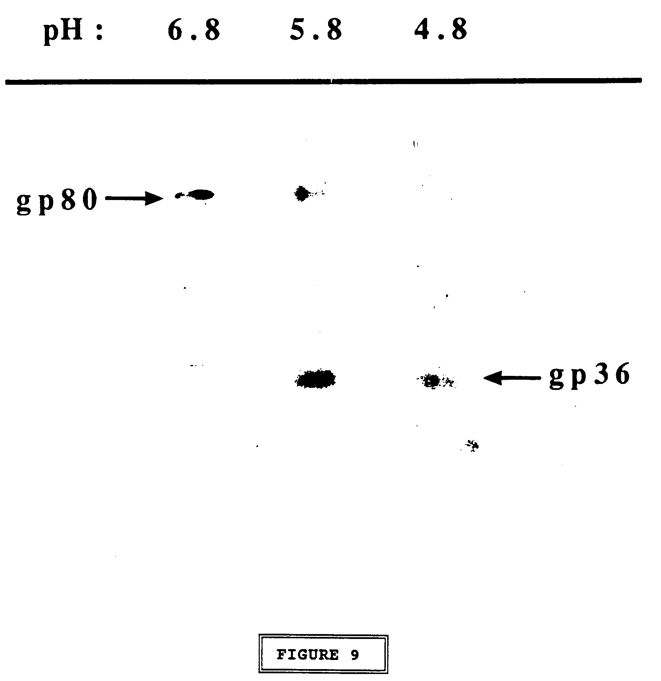

Figure 9:Dissociation de gp80 purifiée en gp36. Des cellules CEM infectées par HIV-2 ont été marquées (17 heures) avec la [³⁵S] méthionine et les agrégats viraux ont été suspensus dans un tampon de lyse contenant du Triton®. Ces extraits viraux (produits correspondant à 2x10⁷ cellules) ont été immunoprécipités en utilisant des anticorps mAb 1H8 et la gp80 a été purifiée par électrophorèse préparative sur gel. Des aliquotes égales de la préparation purifiée de gp80 ont été lyophilisées et resuspendues dans de l'acétate 100mM à pH 6,8, 5,8 et 4,8 contenant 1% (v/v) SDS, 100 unités/ml d'aprotinine et 5mM EGTA (pour inhiber la protéolyse calcium-dépendante). Tous les échantillons ont été incubés à 30°C pendant 60 minutes avant la dilution dans un tampon d'électrophorèse concentré 2 fois. Les échantillons ont été analysés par électrophorèse sur gel de polyacrylamide (12,5%) et on présente un fluorographe.

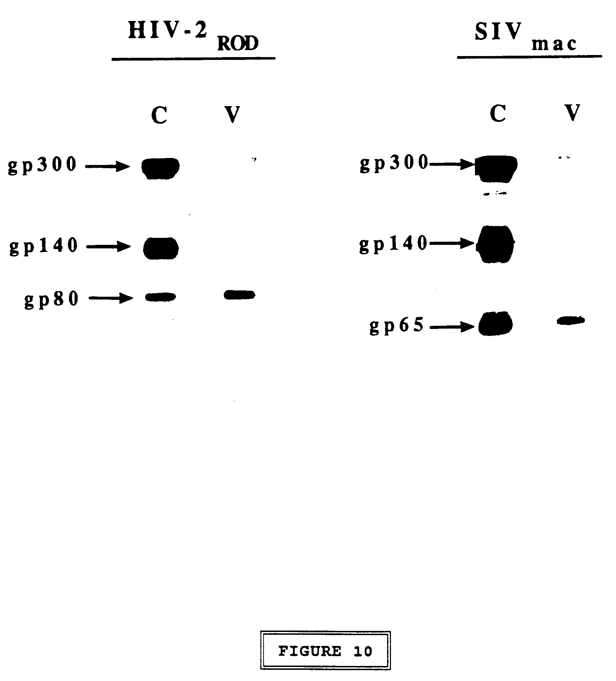

Figure 10:La glycoprotéine transmembranaire de SIV existe sous forme de dimère. Section Cell (cellule): Des cellules HUT-78 infectées par SIV-mac et des cellules CEM infectées par HIV-2 ont été marquées pendant 16 heures avec [³H] glucosamine (200 µCi/ml: 4 x 10⁶ cellules/ml). Les extraits (préparés dans un tampon de lyse contenant du Triton®) des cellules infectées (lignes C) et des agrégats viraux (lignes V) ont été purifiés par immunoprécipitation en utilisant mAb 1H8 et les protéines marquées ont été analysées par électrophorèse sur gel de polyacrylamide 12,5%. On présente le fluorographe.

### EXEMPLES

### MATERIELS ET METHODES

### MATERIELS

La L-[³⁵S]Methionine (activité spécifique >1000 µCi/mmol) L-[6-³H] Fucose (activité spécifique : 45-70 µCi/mmol), D-[6-³H] Glucosamine (activité spécifique : 20-40 µCi/mmol) ont été fournis par Amersham (Amersham, UK). La castanospermine et la tunicamycine ont été fournies par Boehringer-Mannheim (Mannheim, R.F.A.) poly(A).poly(U) provient de l'Institut Curie à Paris et a été préparé selon la méthode décrite par Hovanessian et al -1982- (Journal Interferon Research vol.2 - p.209 à 216).

### VIRUS ET CELLULES

L'isolat HIV-1_{BRU} du virus humain d'immunodeficience de type 1 (12), l'isolat HIV-2_{ROD} du virus humain d'immunodéficience de type 2 (4,5) et le virus Simien d'immunodéficience, SIVmac₁₄₂ (6) ont été utilisés pour ces exemples.

Les différentes lignées cellulaires et lymphocytes humains ont été cultivés dans un milieu de suspension RPMI-1640 (GIBCO-BRL, Cergy-Pontoise France) contenant 10% (v/v) de sérum de veau foetal ; 2µg/ml de polybrene (Sigma) ont été ajoutés pour les cultures de cellules infectées par HIV. Les cellules CEM clone 13 sont dérivées de la lignée de lymphoïdes humains CEM (ATCC-CCL119) et expriment l'antigène T4 à un niveau élevé. Cinq jours après l'infection avec les isolats HIV-1_{BRU} ou HIV-2_{ROD}, environ 80-90% des cellules produisent des particules virales et peuvent être identifiées par l'effet cytopathogène correspondant à la vacuolisation des cellules et par l'apparence de petits syncytiums. La lignée cellulaire HUT-78 est une autre lignée cellulaire de lymphoïdes humains positifs pour le récepteur T4 (Gazdal et al. 1980) qui est hautement permissive pour la réplication de SIVmac₁₄₂ (Daniel et al. 1985). Les lymphocytes du sang périphérique de donneurs sains ont été stimulés pendant 3 jours avec 0,2% (p/v) de fraction P de phytohémagglutinine (Difco, Detroit, USA) dans un milieu RPMI-1640 complété avec 10% de veau foetal. Les cellules ont été mises en culture dans un milieu RPMI-1640 contenant 10% (v/v) de facteur de croissance de cellule T (TCGF, Biotest). Après infection avec HIV-2, les lymphocytes ont été mis en culture en présence de 10% (v/v) TCGF et de 2µg/ml de Polybrene).

### MARQUAGE METABOLIQUE DES CELLULES

Pour le marquage métabolique des protéines, des cellules infectées ont été incubées pendant 16 heures à 37°C dans un milieu de culture minimum MEM (abréviation anglaise de "Minimal Essential Medium") exempt de L-méthionine et de sérum, le milieu étant toutefois complété avec 200µCi/ml[³⁵S]méthionine. Pour le marquage métabolique des glycoprotéines, les cellules infectées ont été incubées pendant 16 heures à 37°C dans un milieu de culture MEM dépourvu de sérum et de glucose mais complété avec 200µCi/ml ³H-Fucose ou 200µCi/ml ³H-glucosamine.

### EXTRAITS CELLULAIRES ET VIRAUX

Les culots cellulaires correspondant à 10⁷ cellules ont été resuspendus dans 100 µl de tampon : 10mM Tris-HCl pH 7,6, 150mM NaCl, 1mM EDTA, 0,2mM PMSF, 100 unités/ml d'aprotinine (Iniprol®, Choay) avant addition de 100µl du même tampon contenant 2% (v/v) de Triton® X-100.

Les extraits cellulaires ont été centrifugés à 12.000g pendant 10 minutes, et le surnageant a été conservé à -80°C jusqu'à utilisation. Pour la préparation des extraits viraux, 100µl de 10X du tampon de lyse (100mM Tris-HCl pH 7,6, 1,5M NaCl, 10mM EDTA, 10%(v/v) Triton X-100, 100 unités/ml d'aprotinine) ont été ajoutés par ml de surnageant clarifié des cellules CEM infectées et traitées comme ci-dessus. Pour la préparation d'extraits à partir d'agrégats de virus, un milieu de culture de cellules infectées a d'abord été centrifugé à 12.000 g pendant 10 minutes avant une centrifugation à grande vitesse à 100.000 g à 15 minutes (centrifugeur TL100 Beckman®). Les agrégats de virus (produit obtenu à partir de 10⁷ cellules) ont ensuite été solubilisés dans 200µl de tampon de lyse.

### PREPARATION D'UN IMMUNOADSORBANT AVEC DES ANTICORPS OBTENUS D'UN PATIENT SEROPOSITIF POUR HIV-2

Les immunoglobulines de sérum d'un patient séropositif pour HIV-2 ont été précipitées avec 50% de (NH₄)₂SO₄, dissous dans 20mM de phosphate de sodium (pH 8,0) et ensuite purifiées sur une colonne de cellulose DEAE (DE52, Whatman®) par élution avec 20mM de phosphate de sodium (pH 8,0). Les immunoglobulines ainsi purifiées ont été jugées pures à 90%. Les anticorps ont ensuite été couplés à une colonne de Sépharose® CL 4B activée avec le CNBr conformément à la technique décrite par Berg (2). Deux miligrammes d'IgG ont été couplés par ml de Sépharose® CL 4B. Dans la suite cet immunoadsorbant sera désigné par "HIV-2 sérum-Sépharose".

### PREPARATION DES PROTEINES DE HIV-2 SUR LA COLONNE D'IMNUNOAFFINITE

Les extraits cellulaires des cellules CEM produisant HIV-2 ont tout d'abord été dilués dans deux volumes de tampon de liaison (20mM Tris-HCl pH 7,6 , 50mM KCl, 150mM NaCl, 1mM EDTA, 20%(v/v) de glycerol, 7mM-βmercaptoethanol, 0,2mM PMSF, 100 unités/ml d'aprotinine) avant incubation avec un volume de HIV-2 sérum-sépharose. Le virus extracellulaire a été traité comme les surnageants des cellules productrices de HIV-2 avec 1/10ème du tampon de liaison concentré 10 fois . L'opération de liaison a été conduite toute une nuit puis la colonne a été lavée suffisamment dans le tampon de liaison. Les protéines liées sur la colonne ont été éluées en procédant à l'ébullition dans un tampon d'électrophorèse (125mM Tris-HCl pH 6,8, 1% (p/v) SDS, 2M d'urée, 20% de glycerol, 0,5% de β-mercaptoethanol). Les protéines éluées ont été récupérées par électrophorèse sur des gels de polyacrylamides SDS 7,5% contenant 6M d'urée et 0,1% de bis-acrylamide à la place de 0,2% (p/v).

### ELECTROPHORESE PREPARATIVE

La récupération des glycoprotéines de HIV-2 (gp300 ou gp80) éluées à partir de la colonne d'affinité été faite par électrophorèse sur gel de polyacrylamide comme décrit précédemment et, les régions du gel contenant les glycoprotéines virales ont été coupées par référence à la position des marqueurs protéiques de poids moléculaire préétablis(BRL).

La glycoprotéine gp300 a été éluée par incubation pendant 16 heures à 4°C dans un tampon d'élution (0,1M NaHCO₃, 0,5mM EDTA, 0,05% (p/v) SDS, 0,2mM PMSF). Les fractions de glycoprotéines ainsi obtenues ont été lyophilisées et conservées au réfrigérateur jusqu'à utilisation. La gp80 ,a été électroéluée dans un tampon contenant 4mM Tris-HCl, pH 7,6 ; 2mM d'acétate de sodium et 2mM d'EDTA

### PREPARATION D'ANTICORPS MURINS POLYCLONAUX DIRIGES CONTRE LA GP300.

La glycoprotéine d'enveloppe de HIV-2 gp300 a été purifiée à partir d'extraits de cellules CEM infectées (3x10⁸ cellules) par chromatographie d'immunoaffinité sur HIV-2 sérum Sépharose® suivi d'une électrophorèse préparative sur gel (13). La préparation purifiée de gp300 a été dissoute dans 10 ml de 150mM NaCl contenant 0,5M d'urée et 1mg/ml de protéines de sérum de souris puis dialysée pendant 24 heures au contact d'une solution contenant 150mMNaCl et 0,5M d'urée. Le produit de dialyse a ensuite été centrifugé et des aliquotes de 2ml ont été conservées à -80°C. Cinq souris (agées de 8 semaines) ont reçu une injection intrapéritonéale,à 5 reprises à 12 jours d'intervalle avec 350 µl de préparation de gp300 (environ 1µg de gp300). Un adjuvant Poly(A).poly(U) (200µg ; 1mg/ml dans 150mM NaCl) a été administré par voie intraveineuse pendant chaque immunisation (17). Cinq jours après la dernière injection les souris ont reçu par injection intrapéritonéale une suspension de 10⁶ cellules de sarcome 180/TG pour préparer un fluide ascite hyperimmun (8). Huit jours après l'injection les souris ont été sacrifiées et les fluides ascites ont été recueillis. Les cellules des ascites ont été enlevées par centrifugation (200g, 5 minutes) et le fluide péritonéal a été recueilli.

### PREPARATION D'ANTICORPS MONOCLONAUX, mAb 1H8

Les virions HIV-2 ont été cultivés dans des cellules CEM et purifiés à partir de surnageants de culture concentrés en formant des bandes, dans des gradients de sucrose. Le virus purifié a été disloqué solubilisé) dans 0,5% Triton®-100, 150mM NaCl, 50mM Tris, pH8,0, 1% d'aprotinine (Sigma®) et clarifié par ultracentrifugation. L'extrait viral a ensuite été passé sur une colonne d'affinité Lentil-Lectin Sepharose® 4B (Pharmacia®), la colonne a été lavée et les glycoprotéines accrochées ont été éluées avec 0,5M méthyl α-D-mannopyranoside (Sigma®) et dialysées toute une nuit au contact d'un tampon salin de phosphate.

Des souris BALB/c ont été immunisées par une injection intrapéritonéale avec 0,3ml de glycoprotéines purifiées 2-5µg, re-accrochées sur Lentil-Lectin Sépharose® 4B (50-100µl). Les souris ont reçu un rappel toutes les 4-6 semaines pendant 24 heures avec le même immunogène. Ces souris ont été suivies pour détecter la production d'anticorps, qualitativement par test RIPA sur des extraits de virions HIV-2 marqués avec [³⁵S] méthionine et quantitativement avec le test EIA sur des virions disloqués (solubilisés) (Genetic System®).

Trois jours après la dernière injection, des cellules de rate ont été fusionnées avec des cellules de myélome NS1 conformément à la méthode de Kohler et Milstein (10). Des plaques de fusion à 96 puits ont été criblées pour détecter la présence d' hybridomes secrétant des anticorps anti-HIV-2 en utilisant le test EIA sur virions disloqués (solubilisés) de Genetic System. Des méthodes pour la propagation et la stabilisation des hybridomes clonés et pour la production d'ascite ont déjà été décrites (7). Les surnageants de culture des hybridomes ont été criblés par des analyses RIPA et Western Blot. Un anticorps monoclonal (mAb)1H8, réagissant avec la glycoprotéine a ensuite été caractérisé avec la séquence d'acides aminés 579-604 de la glycoprotéine transmembranaire de HIV-2 correspondante en utilisant un test EIA basé sur un peptide synthétique (16). La séquence d'acides aminés 579-604 est hautement conservée parmi tous les isolats HIV-2 et SIV séquencés, l'anticorps monoclonal 1H8 produit une réaction croisée avec tous les isolats HIV-2 et SIV ainsi testés. Le peptide synthétique p39' utilisé a été synthétisé d'après la séquence d'acides aminés 579-604 déduite de la séquence nucléotidique de l'enveloppe de HIV-2_{ROD}. La séquence d'acides aminés du peptide synthétique p39' est la suivante:
VTAIEKYLQDQARLNSWGCAFRQVCH

### TEST DE RADIO-IMMUNOPRECIPITATION (RIPA)

Les extraits cellulaires ou viraux (20 µl) (produit correspondant à 10⁶ cellules infectées) ont d'abord été dilués dans deux volumes de tampon RIPA [(10mM Tris-HCl pH 7,6, 150mM NaCl, 1mM EDTA, 1% Triton® X-100(v/v),0,2% sodium déoxycholate (wt/v), 0,1% SDS (p/v), 7mM 2β-mercaptoethanol, 0,2 mM PMSF, 100µ/ml d'aprotinime (Iniprol®,Choay) et 20% de glycérol (v/v)]. Les extraits dilués ont ensuite été incubés (45 minutes, 4°C) avec des anticorps polyclonaux ou monoclonaux (25 µl). La protéine A-Sepharose® a ensuite été ajoutée et les échantillons ont été incubés pendant 3 heures à 4°C. Ces échantillons ont ensuite été lavés dans un tampon RIPA. Les protéines récupérées par immunoprécipitation ont été éluées en chauffant (95°C, 5 minutes) dans un tampon d'électrophorèse [125mM Tris-HCl, pH6,8, 1% SDS (p/v), 20% glycérol (v/v), 0,5% 2 β-mercaptoethanol]. Les protéines éluées ont été récupérées par électrophorèse sur des gels de polyacrylamide SDS 7,5 ou 12,5% contenant 0,1% bis-acrylamide au lieu de 0,2% (p/v).

### ANALYSE IMMUNOBLOT APRES TRANSFERT ELECTROPHORETIQUE : WESTERN BLOT

Les protéines ont été soumises à une analyse par électrophorèse sur gel de polyacrylamide avant d'être électrophorétiquement transférées sur des feuilles de nitrocellulose 0,45µm (Schleicher et Schüll, Dassel, FRG) dans un tampon d'électrode (20mM base Tris 150mM glycine, 20% méthanol, v/v) tel que décrit par (3). Les empreintes d'électrophorèse ont été saturées avec 5% (p/v) de lait en poudre écrémé dans PBS (9). Elles ont ensuite été incubées dans un sac scellé (toute la nuit à 4°C) soit avec des sérums positifs pour HIV-1 ou avec des sérums positifs pour HIV-2 (à une dilution de 1:100) ou avec des anticorps de souris polyclonaux ou monoclonaux (à une dilution de 1:200) dans du PBS contenant 10% FCS. Les feuilles ont ensuite été lavées dans PBS, le PBS contenant 5% de Nonidet P-40 puis resaturée dans PBS contenant du lait écrémé (5%). Les feuilles lavées ont ensuite été incubéeS (2 heures à température ambiante) dans un sac scellé soit avec une préparation de protéines A marquées avec I¹²⁵ (Amersham®, >30mCi/mg) pour révéler les anticorps polyclonaux humains dans les sérums HIV-1 ou HIV-2 ou avec une préparation d'immunoglobulines de chèvre anti-souris marquées au I¹²⁵ (Amersham® ; 2-10 µCi/µg) Les feuilles ont été sorties des sacs et lavées à nouveau, séchées puis autoradiographiées (Kodak® RP Royal, X-Ray Films) pour 24-48 heures.

### RESULTATS

### DETECTION DE LA GLYCOPROTEINE DE 80 kDa DANS DES CELLULES INFECTEES PAR HIV-2 ET DANS LE VIRION

On a déjà montré (13) que le précurseur des glycoprotéines d'enveloppe de HIV-2 est une protéine de 140-kDa (gp140) qui nécessite la formation d'un dimere homologue au cours de sa transformation en produits matures, à savoir les glycoprotéines extracellulaires (gp125) et transmembranaires (gp36). On a maintenant mis en évidence que la glycoprotéine transmembranaire existe sous forme d'un homodimère ayant une mobilité électrophrétique sur des gels de polyacrylamide à une position correspondante à une protéine de 80-kDa (figures 1 à 6). Cette protéine est glycosylée et par commodité elle sera désignée par l'expression gp80.

Des extraits bruts de cellules CEM infectées par HIV-1_{BRU} ou HIV-2_{ROD} ou encore des cellules de ce type non infectées ont été analysés par test immunoblot après transfert électrophorétique (Western blot) en utilisant un sérum positif pour HIV-1 et 3 sérums différents positifs pour HIV-2 recueillis chez des patients atteints de SIDA (figure 1). Les sérums spécifiques de HIV-2 identifiaient les précurseurs de l'enveloppe (gp140 et gp300) et en plus reconnaissaient fortement la glycoprotéine de 80 kDa (gp80). Ces sérums étaient spécifiques des protéines de HIV-2 puisqu'ils ne reconnaissaient pas les protéines de HIV-1 suivantes qui étaient détectables en utilisant un sérum spécifique de HIV-1 : le précurseur de la glycoprotéine d'enveloppe gp120 de HIV-1 et les précurseurs de la protéine gag (p55 et p40 pour HIV-1). L'association de gp80 à l'infection due à HIV-2 a été démontrée par plusieurs résultats dans lesquels gp80 n'a pas été identifié par des sérums HIV-1 positifs ni dans des cellules infectées par HIV-1.

Une analyse Western blot des culots de virus préparés par centrifugation (100.000g pendant 30 minutes) d'un milieu de culture infecté a montré que la gp80 pouvait également être détectée dans des particules de HIV-2 en même temps que la glycoprotéine extracellulaire gp125.

### SYNTHESE DE gp80 DANS DES CELLULES INFECTEES PAR HIV-2

Des expériences préliminaires ont montré que tous les sérums HIV-2 positifs pouvaient immunoprécipiter la gp80 ainsi que les précurseurs de l'enveloppe, gp140 et gp300 ainsi que la glycoprotéine extracellulaire gp125. Pour caractériser la synthèse de gp80, on a utilisé un sérum HIV-2 positif réagissant fortement avec les protéines d'enveloppe. Des anticorps purifiés de ce sérum ont été couplés à une colonne de sépharose® activé par CNBr (HIV-2 sérum-Sépharose), utilisée comme colonne d'immunoaffinité pour purifier les glycoprotéines d'enveloppe. Les cellules infectées par HIV-2 ont été marquées avec [³H]glucosamine et à différents moments (2,3,4,6 et 8 heures) des extraits ont été préparés à partir des cellules infectées et à partir des culots viraux. Tous les échantillons ont été purifiés sur HIV-2 sérum-Sépharose et les protéines marquées ont été analysées par électrophorèse sur gel de polyacrylamide (figure 2); après 2 heures, la gp140 et la gp300 sont les seules protéines marquées détectables dans les cellules infectées. La gp125 et la gp80 sont devenues détectables 3 ou 4 heures après le début du marquage et elles sont également devenues détectables dans les agrégats (culots) de virus préparés à partir du milieu de culture. 6 ou 8 heures après le début du marquage, la gp125 et la gp80 ont pu clairement être détectées. Ces résultats indiquent que la gp80 est associée aux particules virales et suggèrent que cette gp80 doit être un produit mature d'un précurseur qui nécessite une maturation.

Dans ces expériences une gp36 marquée avec le [³H]glucosamine a également pu être détectée mais pas de façon intracellulaire. Une protéine de 200 kDa apparaît également sur la figure 2 et correspond probablement à une protéine cellulaire puisqu'elle n'a pas été immunoprécipitée par d'autres sérums HIV-2 positifs.

Les résultats de cinétique concernant la synthèse des glycoprotéines d'enveloppe de HIV-2 sont conformes aux résultats déjà obtenus. Dans les cellules infectées par HIV-2, la gp140 est le premier produit d'enveloppe détectable 15 minutes après le marquage (pulse-labelling). Pendant la période du suivi du traceur (chase) la forme dimérique du précurseur de l'enveloppe (gp300) a pu être détectée après 30 minutes alors que la glycoprotéine extracellulaire mature (gp125) est devenue détectable 1 h.30 à 3 heures après le début (13).

### IDENTIFICATION DE LA gp80 PAR DES ANTICORPS POLYCLONAUX DIRIGES CONTRE LE PRECURSEUR D'ENVELOPPE DE HIV-2.

Pour caractériser les glycoprotéines d'enveloppe de HIV-2 on a préparé des anticorps polyclonaux dirigés contre le précurseur dimérique purifié gp300. Pour cela la gp300 a d'abord été partiellement purifiée par un immunoadsorbant avec des anticorps d'un patient séropositif pour HIV-2 avant purification par électrophorèse préparative. Cinq souris ont été immunisées avec 5µg de cette préparation de gp300 purifiée, administrée intrapéritonéalement 5 fois à 10 jours d'intervalles. Poly(A).poly(U) (200µg) a été utilisé comme adjuvant administré en mélange avec l'antigène (voir Matériels et Méthodes). Toutes les souris ont développé des anticorps contre la gp300. Ces anticorps sont des anticorps polyclonaux contre la gp300 (anticorps anti-gp300). La figure 3 montre les résultats d'une analyse Western blot mettant en oeuvre les anticorps de l'une des souris immunisées. Des anticorps anti-gp300 ont réagi spécifiquement avec la gp300, mais également avec les gp140, gp125 et gp80 présentes dans des cellules infectées par HIV-2. Aucun signal spécifique n'a été observé dans des cellules CEM infectées par HIV-1 ou dans des cellules non infectées. Le marquage parfois observé d'une protéine de 60 kDa avec des anticorps anti-gp300 était probablement non spécifique puisqu'il a été observé dans les extraits cellulaires indépendamment de l'infection virale (figure 3, section cellules:"CELLS") et dans certaines expériences il n'a pas été observé du tout. Ces anticorps polyclonaux ont également été utilisés dans un test Western blot similaire en mettant en oeuvre des extraits de cellules infectées par HIV-2, de même que des agrégats de virus. Dans les extraits cellulaires, les anticorps reconnaissaient la gp300, la gp140 et la gp80 (figure 3, section HIV-2, ligne C). Dans les extraits viraux, ils reconnaissaient la gp125, la gp80 et la protéine de 36 kDa qui est probablement la glycoprotéine transmembranaire gp36 (figure 3, section HIV-2, ligne V). Après une exposition prolongée il a été possible de détecter un signal correspondant à la position de la gp36 dans les extraits cellulaires. De façon intéressante on note que le niveau de gp80 et de gp36 était beaucoup plus élevé dans l'agrégat (culot) viral comparé à son niveau dans les extraits cellulaires.

Ces résultats montrent que les anticorps polyclonaux dirigés contre le précurseur de la glycoprotéine d'enveloppe identifient la gp80, de même que tous les composants de l'enveloppe de HIV-2. C'est pourquoi la gp80 peut être mise en relation avec l'enveloppe de HIV-2. La glycoprotéine gp80 est associée avec le virus ; il s'agit probablement d'un produit mature.

### IDENTIFICATION DE LA gp80 SPECIFIQUE DE LA GLYCOPROTEINE TRANSMEMBRANAIRE DE HIV-2 PAR DES ANTICORPS MONOCLONAUX 1H8.

L'anticorps monoclonal mAb 1H8 a été utilisé dans un test Western blot pour déterminer quelles protéines virales peuvent être identifiées. Dans les cellules infectées par HIV-2, mAb 1H8 a identifié la gp300, la gp140 et la gp80 alors que dans les particules de HIV-2 il a identifié principalement la gp80 et faiblement la gp36 et la gp300 (figure 4). La présence d'un faible taux de gp300 dans l'agrégat viral était probablement due à des contaminations venant des cellules lysées puisqu'il s'agit d'une protéine cellulaire (13). Le signal faible avec la gp36 reflète probablement le faible niveau de cette protéine. mAb 1H8 n'a pas reconnu la glycoprotéine extracellulaire gp125 (figure 4). De plus, aucune protéine des extraits de cellules infectées par HIV-1 ou de l'agrégat de virus n'a été reconnue. Ces résultats montrent qu'il existe une spécificité de l'anticorps mAb 1H8 pour les précurseurs d'enveloppe de HIV-2 (gp140 et gp300) et la glycoprotéine transmembranaire (gp36). La réactivité de mAb 1H8 a été localisée avec la séquence d'acides aminés 579-604 de la glycoprotéine transmembranaire de HIV-2, en utilisant un peptide synthétique p39'.

Pour montrer la spécificité de la réactivité de mAb 1H8 avec gp80, un test Western blot, utilisant des extraits d'agrégat viral de HIV-2 a été réalisé. Après le transfert des protéines, des feuilles de nitrocellulose ont été incubées avec des anticorps mAb 1H8 ou des anticorps polyclonaux contre la gp300, en l'absence ou en présence de peptides 39' (figure 5). Le mAb 1H8 a donné un signal fort avec la gp80. De plus, un signal pour la gp36 a été observé mais uniquement après une exposition prolongée de l'autoradiogramme. Des anticorps anti-gp300 ont réagi avec la gp125, la gp80 et la gp36 ; le signal obtenu avec la protéine 60-kDa n'était pas spécifique (comme dans la figure 3). L'addition de peptide p39' a complètement aboli les signaux obtenus avec mAb 1H8 mais pas ceux obtenus avec les anticorps anti-gp300 (figure 5). Ces observations confirment que la réactivité de mAb 1H8 pourrait être spécifique du résidu de 26 acides aminés correspondant à la séquence 579 à 604 de la glycoprotéine transmembranaire de HIV-2. En conséquence, une séquence correspondant à celle du peptide p39' pourrait être présente dans la gp80. La réactivité des anticorps anti-gp300 n'a pas été modifiée par le peptide P39'. C'est pourquoi ces anticorps polyclonaux pourraient interagir avec d'autres épitopes que ceux correspondant au peptide p39'.

### IMMUNOPRECIPITATION DE LA gp80 PAR DES ANTICORPS POLYCLONAUX ANTI-gp300 ET PAR mAb 1H8

Des anticorps polyclonaux ont immunoprécipité la gp300, la gp140 et la gp125 ainsi que la gp80, alors que mAb 1H8 a immunoprécipité la gp300, la gp140 et la gp80 (figure 6). Deux protéines cellulaires (60 et 45-kDa) ont également été associées avec les préparations de complexes immuns en utilisant à la fois les anticorps polyclonaux et monoclonaux (figure 6, lignes 0 et 2 heures). La présence de protéines de 60 et 45 kDa dans la préparation de complexes immuns était due à leur accrochage avec la protéine A Sepharose® ; cette dernière a été utilisée de façon à récupérer les complexes immuns formés avec les différents anticorps.

Des cellules infectées par HIV-2 ont été marquées pendant 30 minutes par la technique "pulse" et le marqueur a été suivi (chase) pendant 2 et 4 heures. Des extraits de cellules marquées (temps 0,2 et 4 heures) et l'agrégat viral récupéré après 4 heures de suivi (chase) ont été analysés par test d'immunoprécipitation en utilisant des anticorps polyclonaux contre la gp300 et l'anticorps mAb 1H8 (figure 6). Avec les anticorps polyclonaux et monoclonaux, la gp80 ne pouvait être détectée pendant la période de marquage (pulse). Elle est devenue clairement apparente après 2 heures de suivi (chase) dans les cellules infectées. Lorsque le suivi (chase) a été prolongé jusqu'à 4 heures, alors la gp80 marquée à la [³⁵S]méthionine est devenue indétectable dans les cellules infectées. L'analyse de l'agrégat viral produit après 4 heures a montré que, comme la glycoprotéine extracellulaire gp125, la gp80 était associée aux particules virales (figure 6). On peut penser que la gp80 est un produit de maturation de l'enveloppe de HIV-2. Le fait que la gp80 a été identifiée par des anticorps monoclonaux spécifiques de la glycoprotéine transmembranaire de HIV-2 a montré qu'elle correspond à la forme dimérique de la gp36 (confirmé par les résultats montrés sur les figures 8 et 9). La détection de gp80 n'était pas restreinte aux cellules CEM infectées puisqu'elle a également été détectée dans les lymphocytes T4 infectés par HIV-2.

La comparaison des résultats obtenus par des analyses Western blot et par des tests d'immunoprécipitation montre que les sérums humains positifs pour HIV-2, les anticorps polyclonaux murins et mAb 1H8 reconnaissent les formes dénaturées de gp80 et gp125 mieux que les formes natives (figure 1, 2, 3, 4 et 6). Les formes natives de ces glycoprotéines matures ont probablement des conformations qui masquent les épitopes identifiés par les différents anticorps.

### INCORPORATION DE GLUCOSAMINE ET DE FUCOSE DANS LA gp80

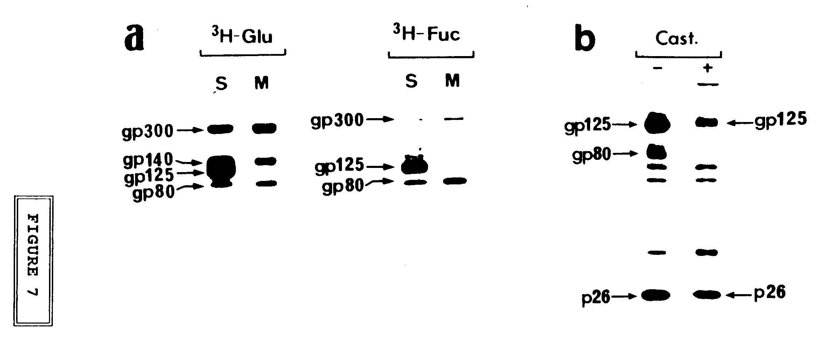

Des extraits de cellules infectées par le virus HIV-2, métaboliquement marquées avec ^{[3}H]glucosamine et [³H]fucose, ont été immunoprécipités en utilisant des anticorps mAb 1H8 et des anticorps polyclonaux anti-gp300. Conformément aux résultats précédents (figures 3-6) les anticorps anti-gp300 ont immunoprécipité des glycoprotéines marquées avec les sucres, à savoir les gp300, gp140, gp125 et gp80 alors que mAb 1H8 a immunoprécipité les gp300, gp140 et gp80. Toutes ces protéines ont incorporé le [³H]glucosamine alors que l'incorporation de [³H]fucose apparaissait la plupart du temps dans les gp125 et gp80 (figure 7A). Dans ces expériences, le marquage de la gp36 avec [³H]fucose a été observé faiblement après une exposition prolongée de l'autoradiogramme. La gp80 peut aussi incorporer du [³H]mannose de la même façon que les gp300, gp140 et gp125. L'incorporation de ces sucres marqués dans la gp80 et dans d'autres glycoprotéines a été totalement bloquée avec de la tunicamycine (15) un antibiotique qui inhibe la glycosylation des protéines liées sur l'azote.

Les oligosaccharides de glycoprotéines, liés sur l'asparagine (contenant la N-acétylglucosamine, le mannose et le glucose) subissent une maturation plus importante après leur attachement à des protéines natives (11). Les chaînes oligosaccharidiques sont coupées aux extrémités dans le réticulum endoplasmique et dans l'appareil de Golgi avant le transfert des résidus fucose et de l'acide sialique. C'est pourquoi l'incorporation des résidus fucose a lieu longtemps après les étapes de glycosylation. Dans les cellules infectées par HIV-2, le [³H]fucose est incorporé en majorité dans la gp125 et la gp80 (figure 7) deux protéines qui correspondent à des produits matures de précurseurs d'enveloppe de HIV-2 (figure 6). Pour confirmer que la gp120 a été produite pendant la maturation du précurseur d'enveloppe, des cellules infectées avec HIV-2 ont été marquées avec la [³⁵S]méthionine en l'absence ou en présence d'un inhibiteur de glucosidase, la castanospermine (14). Les surnageants de culture ont ensuite été testés par immunoprécipitation en utilisant un sérum d'un patient positif pour HIV-2 qui reconnaissait plusieurs protéines virales. En présence de castanospermine, la production de gp80 et de gp125 a été considérablement réduite alors que la production de la protéine majeure de nucléocapside (core) (p26) n'a pas été significativement affectée (figure 7b).

### DISSOCIATION DE gp80 EN gp36

Des expériences ont été réalisées pour optimiser les conditions dans lesquelles la dissociation de gp80 pouvait avoir lieu, ces conditions correspondant à un milieu fortement salin, un pH acide, un détergent ionique EDTA ou EGTA. Nous avons testé la méthode telle que décrite dans (13) permettant de dissocier la gp300 par incubation dans un tampon faiblement acide. Bien que cette méthode puisse convenir à la dissociation de gp80, avec un tampon à pH inférieur à pH6, la plupart des glycoprotéines gp80 ont été dégradées. Dans toutes les expériences on a préparé des extraits de cellules infectées ou d'agrégats viraux en mettant en oeuvre un tampon de lyse contenant un détergent non ionique, le Triton® X-100. Dans ces conditions, la gp300 et la gp80 ne sont pas dissociées, même après addition de détergent ionique SDS. On a recherché l'effet du SDS lorsqu'il est substitué au Triton® pour la préparation des extraits. Les cellules infectées par HIV ont été marquées avec la [³⁵S]métionine avant préparation des extraits par solubilisation dans un tampon de lyse contenant soit 1% de Triton® X-100 soit 1% de SDS. Ces extraits ont ensuite été dilués 10 fois dans un tampon de lyse sans détergent et immunoprécipités en utilisant mAb 1H8. Les préparations de complexes immuns à partir des extraits au Triton® ont montré la présence de bandes marquées à la [³⁵S]méthionine correspondant à la gp300, la gp140 et la gp80, et des bandes faibles de gp36 (figure 8, section C ligne 1). Par ailleurs, lorsque les extraits ont été préparés avec le SDS, alors la gp300 et la gp80 étaient pratiquement indétectables alors que le niveau de gp140 et de gp36 étaient accrus (figure 8, section C, ligne 2). Donc en présence d'un détergent ionique les formes dimériques gp300 et gp80 ont été dissociées en donnant lieu à la production de gp140 et de gp36 respectivement. La dissociation de gp300 purifiée, marquée à la [³⁵S]méthionine a donné lieu seulement à la formation de gp140 (13). La gp36 ne doit donc exister qu'après la dissociation de la gp80. On remarque que la dégradation de protéines peut également avoir lieu en présence de SDS dès lors que toutes les protéines gp300 et gp80 marquées n'ont pas été récupérées parmi les protéines dissociées (figure 8 section C). La présence de 200 unités/ml d'aprotinine et de 0,2mM PMSF n'a pas empêché une telle dégradation pendant l'incubation avec SDS. Il se peut que les formes dimériques des protéines présentent une conformation telles qu'elles résistent à la protéolyse. La dissociation de gp300 et gp80 peut ensuite conduire à des modifications conformationnelles rendant les sites protéolytiques accessibles.

Les expériences de dissociation ont également été menées avec des agrégats viraux de HIV-2. Les protéines virales marquées à la [³⁵S]méthionine ont été solubilisées dans un tampon de lyse contenant 1% de Triton® ou 1% de SDS dans un tampon RIPA contenant 0,1% de SDS et 1% de déoxycholate. Les extraits dans le tampon de lyse contenant 1% de SDS ont également été chauffés à 95°C. Tous les extraits ont ensuite été immunoprécipités avec mAb 1H8 et analysés par électrophorèse sur gel de polyacrylamide (figure 8 section V).

Dans le complexe immun préparé à partir des extraits au Triton®, la gp80 et la gp36 étaient les principales protéines immunoprécipitées par l'anticorps 1H8. Par ailleurs, la gp80 est devenue indétectable alors que le niveau de gp36 était sensiblement accru dans les extraits au SDS. Le degré de dégradation des extraits au SDS était probablement particulièrement important puisque la plupart des constituants marqués ont disparu. Un résultat meilleur a été obtenu avec le tampon RIPA dans lequel la plupart des gp80 ont été dissociées et environ 30% du marquage a été retrouvé sur les gp36 (figure 8 section V). Pour montrer que la gp80 est composée uniquement de gp36 des expériences de dissociation ont été conduites en utilisant la gp80 marquée à la [³⁵S]méthionine purifiée par immunoprécipitation avec les anticorps mAb 1H8 et par électrophorèse préparative sur gel. Les préparations de gp80 lyophilisées ont ensuite été suspendues directement dans un tampon acétate contenant du SDS, à pH 6,8, 5,8 et 4,8. A pH 4,8 toutes les gp80 étaient converties en gp36 (figure 9).

### CARACTERISATION D'UNE GLYCOPROTEINE TRANSMEMBRANAIRE SOUS FORME DIMERIQUE CHEZ SIV-mac

On a recherché si la glycoprotéine transmembranaire était détectable sous forme de dimère dans des échantillons SIV.

Des cellules infectées par SIV et HIV-2 ont été marquées avec [³H]glucosamine et des extraits ont été préparés dans un tampon de lyse contenant du Triton® puis immunoprécipités avec l'anticorps mAb 1H8. Comme le montre la figure 7, l'anticorps monoclonal a précipité les gp300, gp140 et gp80 des cellules infectées par HIV-2, alors que seulement la gp80 a été trouvée dans les particules virales (figure 10). Dans les cellules infectées par SIV, l'anticorps monoclonal a précipité 3 protéines glycosylées : le précurseur d'enveloppe gp140, le précurseur dimérique gp300 et une protéine de 65 kDa (gp65) qui correspond probablement à la contrepartie de la gp80 de HIV-2. Comme la gp80, la gp65 est apparue associée avec les particules virales de SIV (figure 10).

On remarque que dans ces expériences, les formes monomériques de la glycoprotéine transmembranaire de HIV-2_{ROD} et SIV-mac n'étaient pas détectables. La séquence d'acides aminés 579-604 de HIV-2_{ROD} correspond à la séquence d'acides aminés 595-620 de SIV-mac (19,7). Ces deux séquences présentent une forte homologie et l'anticorps mAb 1H8 réagit de façon croisée avec les protéines d'enveloppe à la fois de HIV-2_{ROD} et de SIV-mac.

Les inventeurs ont donc maintenant mis en évidence qu'une protéine d'un poids moléculaire d'environ 65 kDa correspond à une forme dimérique de la gp32 du virus SIV-mac.

### DISCUSSION

La dissociation des formes dimériques (gp300 et gp80) peut avoir lieu à pH légèrement acide. Conformément à ce résultat on peut supposer que la dimérisation de gp140 peut être dépendante du pH et avoir lieu dans un compartiment du réticulum endoplasmique qui favorise la fusion de 2 molécules du précurseur gp140.

Le poids moléculaire de la forme dimérique du précurseur d'enveloppe gp300 de HIV-2 a été estimé par électrophorèse sur gel de polyacrylamide dans les conditions dénaturantes (13). Dans un gel de polyacrylamide 5%, contenant 0,1% de bisacrylamide au lieu de 0,2% (p/v), ce précurseur dimérique a migré à une position correspondant à une protéine de 280-kDa. Pour confirmer le poids moléculaire du dimère dans les conditions natives, des expériences de filtration sur gel en utilisant une colonne Séphacryl® S-300, et des extraits de cellules infectées par HIV-2 marquées à la [³⁵S]méthionine préparés sur un tampon de lyse contenant du Triton, ont été réalisées. Dans ces conditions expérimentales, la gp300 a été éluée sous la forme d'un second pic après le pic des protéines agrégées. Le dimère de glycoprotéines transmembranaires correspond à une protéine éluée ayant un poids moléculaire de 75-80 kDa après le pic de la gp125 et avant le pic de la sérum-albumine bovine (68 kDa) utilisée comme marqueur. Ces observations montrent que les estimations de poids moléculaire des dimères natifs et dénaturés gp300 et gp80 sont comparables. In vitro la dissociation de ces formes dimériques a lieu à pH acide et également en présence d'un détergent ionique SDS. Lorsque les extraits ont été préparés dans le tampon de lyse contenant du Triton®, les formes dimériques gp300 et gp80 ont résisté à la dissociation par SDS. Le détergent non ionique Triton® conserve donc les formes natives des dimères de l'enveloppe gp300 et gp80.

Les formes dimériques du précurseur d'enveloppe gp300 et de la glycoprotéine transmembranaire ont également été observées dans les cellules infectées par SIV mais pas par HIV-1. De façon particulière les dimères transmembranaires de HIV-2 (gp80) et SIV (gp65) résistent à la dissociation par des agents réducteurs. C'est pourquoi la dimérisation de la glycoprotéine transmembranaire d'enveloppe peut être considérée comme une propriété spécifique de l'expression des gènes d'enveloppe de HIV-2 et SIV. Cette propriété peut être utilisée pour la caractérisation de nouveaux isolats HIV, à titre de marqueur adapté pour décrire la relation de l'isolat détecté avec au type HIV-1 ou au type HIV-2. La dimérisation du précurseur d'enveloppe pourrait être indispensable pour sa maturation afin de produire des glycoprotéines d'enveloppe matures. Alors que la forme dimérique de la glycoprotéine transmembranaire peut être essentielle pour la formation optimale de la structure du virion et ainsi pour sa capacité à fusionner avec les membranes cellulaires et à être infectieux, la dissociation in vitro du dimère transmembranaire conduit à une dégradation très importante. Ceci peut être dû à la capacité de la forme dimérique de cette glycoprotéine de résister à la protéolyse grâce à sa conformation.

Le tableau ci-après et le schéma des étapes d'obtention des glycoprotéines gp125 et gp80 de HIV-2 résument certains aspects de l'invention exposés dans les exemples.

| GLYPROTEINES D'ENVELOPPE DE HIV-1, HIV-2 et SIV | | | |
|---|---|---|---|
| GLYCOPROTEINE D'ENVELOPPE | HIV-1_{Bru} | HIV-2_{ROD} | SIV_{MAC} |
| Précurseur | gp160 | gp140 | gp140 |
| Dimère Précurseur | aucun | gp300 | gp300 |
| Extracellulaire | gp120 | gp125 | gp130 |
| Transmembranaire | gp41 | gp36 | gp32 |
| Dimère Transmembranaire | aucun | gp80 | gp75 |

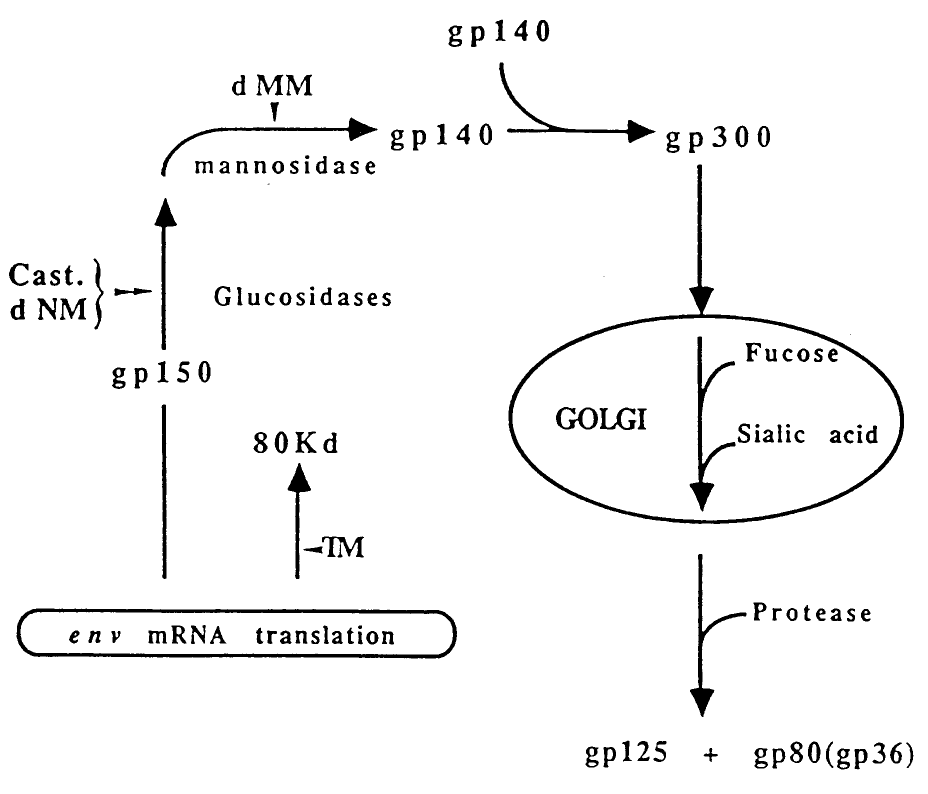

### BIBLIOGRAPHIE

1 - Barré-Sinoussi, F. et al., 1983.
   Science 220.868-871.
2 - Berg, K., 1977.
   J. Immunol. 6, 77-86.
3 - Burnette, W.N. 1981.
   Anal. Biochem. 112, 195-230.
4 - Clavel F. et al. 1986a.
   Science 233, 343-346.
5 - Clavel, F. et al. 1986b.
   Nature 324 : 691-694.
6 - Daniel, M.D. et al. 1985. Science 228, 1201-1204.
7 - Gosting, L., et al. 1987. Journal of Clinical Microbiology, 25, 845-848.
8 - Hovanessian, A.G., et al. 1988. Immunology Today 9, 161-162.
9 - Johnson, D.A. et al. 1984. Gene Anal. Techn. 1, 3-8.
10 - Kohler, G., and Milstein, C. 1975. Nature, 256, 495-497.
11 - Kornfeld, R. et al. 1985. Ann. Rev. Biochem. 54, 632-664.
12 - Montagnier, L. et al. 1984. Cold Spring Harbor Laboratory New York, 1984, pp 363-379.
13 - Rey, M.A. et al. 1989. J. Virol. 63, 647-658.
14 - Saul. R., et al. 1983. Arch. Biochem. Byophys. 221, 593-597.
15 - Schwartz, R.T. et al. 1976. J. Virol. 19, 782-791.
16 - Shriver, K.,et al. 1988, Medical Virology VII, pp 209-224.
17 - Veronese, F.D.M. et al. 1989. J. Virology 63, 1416-1319.
18 - Weiss R.A. 1988. Nature 331, 15.
19 - Chakrabarti, L. et al. 1987. Nature 328, 543.547.

## Revendications

1. Glycoprotéine caractérisée par les propriétés suivantes :
- elle est formée à partir d'un dimère d'une glycoprotéine transmembranaire d'enveloppe d'un rétrovirus HIV ou SIV,
- elle est capable de former un complexe immunologique du type antigène-anticorps, avec des anticorps dirigés contre une glycoprotéine antigénique gp80 ayant un poids moléculaire voisin de 80 kDa, reconnue par des anticorps polyclonaux dirigés contre la glycoprotéine gp300 d'un rétrovirus humain HIV-2, et comprenant en association sous forme d'un dimère, deux entités de la glycoprotéine transmembranaire d'enveloppe gp36 codée par le génome d'un rétrovirus humain du type HIV-2, susceptible de provoquer un SLA ou un SIDA chez l'homme,
- elle a un poids moléculaire du même ordre que celui de la susdite gp80 de HIV-2

2. Glycoprotéine antigénique gp80 selon la revendication 1, caractérisée par les propriétés suivantes :
- elle a un poids moléculaire (PM) voisin de 80 kDa,
- elle est reconnue par des anticorps polyclonaux dirigés contre la glycoprotéine gp300 d'un rétrovirus humain HIV-2,
- elle comprend en association sous forme d'un dimère, deux entités de la glycoprotéine transmembranaire d'enveloppe gp36 codée par le génome d'un rétrovirus humain du type HIV-2, susceptible de provoquer un SLA ou un SIDA chez l'homme .

3. Glycoprotéine gp80 selon la revendication 2, caractérisée en ce que :
- elle peut être associée à des cellules infectées par un rétrovirus humain du type HIV-2 ou/et à des virions ou/et à des virus du type HIV-2,
- elle n'est pas dissociée in vitro en présence de détergent non ionique tel que le Triton ®X-100 1% et elle est dissociée in vitro pour former la glycoprotéine transmembranaire gp36 d'un rétrovirus du type HIV-2, en présence de détergent ionique tel que le SDS 1% à pH faiblement acide.

4. Protéine dérivée d'une glycoprotéine gp80 selon l'une quelconque des revendications 1 à 3, caractérisée en ce qu'elle est dépourvue des groupes glycosylés de la gp80.

5. Anticorps polyclonal caractérisé,
- en ce qu'il reconnaît spécifiquement d'une part, la gp300, la gp140, la gp125 et la gp80 selon l'une quelconque des revendications 1 à 4, présentes dans des cellules infectées par un rétrovirus du type HIV-2, d'autre part, la gp125, la gp80 selon l'une quelconque des revendications 1 à 4 et la gp36 présentes dans des extraits de rétrovirus du type HIV-2 et,
- en ce qu'il ne reconnaît pas les protéines ou glycoprotéines de cellules saines ou de cellules infectées par un rétrovirus du type HIV-1.

6. Anticorps monoclonal caractérisé en ce qu'il réagit spécifiquement avec la glycoprotéine gp80 selon l'une quelconque des revendications 1 à 4 en ce qu'il ne réagit pas avec les protéines et glycoprotéines de cellules infectées par un rétrovirus du type HIV-1 ou avec des agrégats viraux de HIV-1 et en ce qu'il reconnaît en outre les glycoprotéines gp300, gp140 et gp36 de HIV-2.

7. Hybridome producteur d'anticorps monoclonaux selon la revendication 6.

8. Composition antigénique pour le diagnostic spécifique in vitro d'une infection due à un rétrovirus humain du type HIV-2, dans un échantillon biologique susceptible de contenir des anticorps formés après ladite infection, caractérisée en ce qu'elle comprend au moins la glycoprotéine gp80 selon l'une quelconque des revendications 1 à 4.

9. Composition immunogène caractérisée en ce qu'elle comprend au moins la glycoprotéine gp80 selon l'une quelconque des revendications 1 à 4, en association avec un véhicule pharmaceutique acceptable pour la constitution de vaccins.

10. Procédé pour le diagnostic in vitro d'une infection due spécifiquement à un rétrovirus du type HIV-2 caractérisé par les étapes de :
- mise en contact d'un échantillon biologique susceptible de contenir des anticorps produits à la suite d'une infection par un rétrovirus du type HIV-2, avec une glycoprotéine gp80 selon l'une quelconque des revendications 1 à 4 ou avec une composition antigénique selon la revendication 8, dans des conditions appropriées permettant la formation d'un complexe immunologique du type antigène-anticorps, et
- détection du complexe antigène-anticorps éventuellement formé.

11. Procédé de préparation de glycoprotéine antigénique gp80 selon l'une quelconque des revendications 1 à 4, caractérisé par les étapes suivantes :
- lyse de cellules infectées par un rétrovirus humain HIV-2 et séparation du surnageant et des cellules infectées ou lyse des culots viraux préparés par centrifugation,
- incubation de l'extrait cellulaire et/ou de l'extrait viral sur une colonne d'immunoaffinité, sur laquelle est déposé un immunoadsorbant renfermant des anticorps purifiés, tels qu'obtenus à partir d'un sérum d'un malade infecté par le rétrovirus humain HIV-2, ce sérum ayant la capacité de réagir fortement avec les protéines d'enveloppe de HIV-2, lesdits anticorps étant fixés avantageusement sur un support adapté, en présence d'un tampon et pendant un temps suffisamment long pour obtenir la formation d'un complexe immunologique antigène-anticorps,
- lavage de la colonne avec un tampon pour éliminer les molécules non retenues sur le support, et
- récupération des protéines antigéniques recherchées.

12. Procédé selon la revendication 11 pour la préparation de la glycoprotéine selon l'une quelconque des revendications 1 à 4, caractérisé en ce que la récupération des glycoprotéines est faite par électrophorèse et par électroélution.

13. Procédé selon la revendication 11 pour la préparation de la glycoprotéine selon l'une quelconque des revendications 1 à 4, caractérisé en ce que la récupération des glycoprotéines est obtenue par
- élution des protéines fixées sur la colonne d'immuno-affinité ci-dessus, et
- purification des produits ainsi élués sur une colonne de chromatographie contenant, fixés sur le support de séparation des anticorps monoclonaux reconnaissant la gp80.

14. Nécessaire ou kit pour la détection d'anticorps dans le sérum ou dans un autre échantillon biologique d'un patient susceptible d'être infecté par un rétrovirus humain HIV-2, caractérisé en ce qu'il comprend :
- au moins une protéine ou/et une glycoprotéine antigénique selon l'une quelconque des revendications 1 à 4,
- ou une composition selon la revendication 8,
- ou encore un mélange de ces différents constituants et
- des moyens permettant la réaction de formation du complexe immunologique entre les antigènes ci-dessus et les anticorps éventuellement présents dans l'échantillon biologique à tester, notamment si besoin un ou plusieurs tampons d'incubation,
- un échantillon de contrôle négatif, et
- des moyens pour la révélation du complexe antigène-anticorps formé.

15. Procédé pour la détection dans un échantillon biologique d'une infection par le rétrovirus HIV-2, caractérisé par les étapes de :
- traitement de l'échantillon biologique de façon à exposer les protéines intracellulaires, et
- détection de la présence de la glycoprotéine selon la revendication 1 parmi les protéines intracellulaires exposées, par exemple par électrophorèse ou par un test immunologique au moyen d'anticorps immunologiquement réactifs avec la gp80.

## Claims

1. Glycoprotein characterized by the following properties:
- it is formed from an HIV or SIV retrovirus envelope transmembrane glycoprotein dimer,
- it is capable of forming an antigen-antibody type immunological complex with antibodies directed against an antigenic glycoprotein gp80 having a molecular weight close to 80 kDa, recognized by polyclonal antibodies directed against the glycoprotein gp300 of a human retrovirus HIV-2, and comprising in association in the form of a dimer two components of the envelope transmembrane glycoprotein gp36 encoded by the genome of an HIV-2 type human retrovirus capable of causing LAS or AIDS in man,
- it has a molecular weight of the same order as that of the abovementioned HIV-2 gp80.

2. Antigenic glycoprotein gp80 according to Claim 1, characterized by the following properties:
- it has a molecular weight (MW) close to 80 kDa,
- it is recognized by polyclonal antibodies directed against the glycoprotein gp300 of a human retrovirus HIV-2,
- it comprises in association in the form of a dimer two components of the envelope transmembrane glycoprotein gp36 encoded by the genome of an HIV-2 type human retrovirus capable of causing LAS or AIDS in man.

3. Glycoprotein gp80 according to Claim 2, characterized in that:
- it may be associated with cells infected by an HIV-2 type human retrovirus or/and with virions or/and with HIV-2 type viruses,
- it is not dissociated in vitro in the presence of a nonionic detergent such as 1% Triton® X-100 and it is dissociated in vitro in order to form the transmembrane glycoprotein gp36 of an HIV-2 type retrovirus, in the presence of an ionic detergent such as 1% SDS at a weakly acidic pH.

4. Protein derived from a glycoprotein gp80 according to any one of Claims 1 to 3, characterized in that it lacks the glycosylated groups of gp80.

5. Polyclonal antibody characterized in that,
- it recognizes specifically, on the one hand, gp300, gp140, gp125 and gp80 according to any one of Claims 1 to
4, which are present in cells infected by an HIV-2 type retrovirus, and on the other hand, gp125, gp80 according to any one of Claims 1 to 4 and gp36 which are present in extracts of an HIV-2 type retrovirus, and
- it does not recognize the proteins or glycoproteins of healthy cells or of cells infected by an HIV-1 type retrovirus.

6. Monoclonal antibody characterized in that it reacts specifically with the glycoprotein gp80 according to any one of Claims 1 to 4, in that it does not react with the proteins and glycoproteins of cells infected by an HIV-1 type retrovirus or with viral aggregates of HIV-1 and in that it recognizes, in addition, the HIV-2 glycoproteins gp300, gp140 and gp36.

7. Hybridoma which produces monoclonal antibodies according to Claim 6.

8. Antigenic composition for the specific in vitro diagnosis of an infection due to an HIV-2 type human retrovirus, in a biological sample capable of containing antibodies formed after the said infection, characterized in that it comprises at least the glycoprotein gp80 according to any one of Claims 1 to 4.

9. Immunogenic composition characterized in that it comprises at least the glycoprotein gp80 according to any one of Claims 1 to 4, in association with a pharmaceutically acceptable vehicle for the preparation of vaccines.

10. Process for the in vitro diagnosis of an infection due specifically to an HIV-2 type retrovirus, characterized by the steps of:
- bringing a biological sample capable of containing antibodies produced following an infection by an HIV-2 type retrovirus into contact with a glycoprotein gp80 according to any one of Claims 1 to 4 or with an antigenic composition according to Claim 8, under appropriate conditions allowing the formation of an antigen-antibody type immunological complex, and
- detecting the antigen-antibody complex which may be formed.

11. Process for the preparation of an antigenic glycoprotein gp80 according to any one of Claims 1 to 4, characterized by the following steps:
- lysis of cells infected with a human retrovirus HIV-2 and separation of the supernatant and of the infected cells or lysis of the viral pellets prepared by centrifugation,
- incubation of the cellular extract and/or of the viral extract on an immunoaffinity column on which is deposited an immunoadsorbent containing purified antibodies as obtained from a serum from a patient infected with the human retrovirus HIV-2, this serum having the capacity to react strongly with the HIV-2 envelope proteins, the said antibodies being advantageously attached to a suitable support, in the presence of a buffer and for a sufficiently long period to obtain the formation of an antigen-antibody immunological complex,
- washing of the column with a buffer to remove the molecules not retained on the support, and
- recovery of the desired antigenic proteins.

12. Process according to Claim 11, for the preparation of the glycoprotein according to any one of Claims 1 to 4, characterized in that the recovery of the glycoproteins is made by electrophoresis and by electroelution.

13. Process according to Claim 11 for the preparation of the glycoprotein according to any one of Claims 1 to 4, characterized in that the recovery of the glycoproteins is obtained by
- elution of the proteins attached to the above immunoaffinity column, and
- purification of the products thus eluted on a chromatographic column containing, attached to the separation support, monoclonal antibodies which recognize gp80.

14. Set or kit for the detection of antibodies in the serum or in another biological sample from a patient who may be infected with a human HIV-2 retrovirus, characterized in that it comprises:
- at least one protein or/and one antigenic glycoprotein according to any one of Claims 1 to 4,
- or a composition according to Claim 8,
- or alternatively a mixture of these different constituents and
- means permitting the reaction for the formation of the immunological complex between the above antigens and the antibodies which may be present in the biological sample to be tested, especially, if required, one or more incubation buffers,
- a negative control sample, and
- means for visualizing the antigen-antibody complex formed.

15. Process for the detection, in a biological sample, of an infection by the HIV-2 retrovirus, characterized by the steps of:
- treating the biological sample so as to expose the intracellular proteins, and
- detecting the presence of the glycoprotein according to Claim 1 among the intracellular proteins exposed, for example by electrophoresis or by an immunological test by means of antibodies immunologically reactive with gp80.

## Patentansprüche

1. Glykoprotein,
gekennzeichnet durch die folgenden Eigenschaften:
- es wird aus einem Dimer eines Transmembranglykoproteins der Hülle eines Retrovirus HIV oder SIV gebildet,
- es ist in der Lage, einen immunologischen Komplex vom Antigen-Antikörper-Typ zu bilden mit Antikörpern, die gegen ein antigenes Glykoprotein gp80 mit einem Molekulargewicht von ungefähr 80 kDa gerichtet sind, das von polyklonalen, gegen das Glykoprotein gp300 eines humanen Retrovirus HIV-2 gerichteten Antikörpern erkannt wird und das in Assoziation in dimerer Form zwei Einheiten des Transmembranglykoproteins der Hülle gp36 enthält, für das das Genom eines humanen Retrovirus des Typs HIV-2, das ein LAS oder ein AIDS beim Menschen hervorrufen kann, kodiert,
- es hat ein Molekulargewicht derselben Größenordnung wie jenes des obengenannten gp80 von HIV-2.

2. Antigenes Glykoprotein gp80 nach Anspruch 1,
gekennzeichnet durch die folgenden Eigenschaften:
- es hat ein Molekulargewicht (MG) von ungefähr 80 kDa,
- es wird von polyklonalen, gegen das Glykoprotein gp300 eines humanen Retrovirus HIV-2 gerichteten Antikörpern erkannt,
- es umfaßt in Assoziation in dimerer Form zwei Einheiten des Transmembranglykoproteins der Hülle gp36, für das das Genom eines humanen Retrovirus des Typs HIV-2, das ein LAS oder ein AIDS beim Menschen hervorrufen kann, kodiert.

3. Glykoprotein gp80 nach Anspruch 2,
dadurch gekennzeichnet, daß
- es mit durch ein humanes Retrovirus des Typs HIV-2 infizierten Zellen und/oder Virionen und/oder Viren des Typs HIV-2 assoziiert sein kann,
- es in vitro in Gegenwart eines nichtionischen Detergens, wie 1 % Triton^{(R)} X-100, nicht dissoziiert ist und es in vitro in Gegenwart eines ionischen Detergens, wie 1 % SDS, bei einem schwach sauren pH dissoziiert ist, um das Transmembranglykoprotein gp36 eines Retrovirus des Typs HIV-2 gp36 zu bilden.

4. Von einem Glykoprotein gp80 nach einem der Ansprüche 1 bis 3 abgeleitetes Protein,
dadurch gekennzeichnet, daß es von Glykosylgruppen des gp80 befreit ist.

5. Polyklonaler Antikörper,
gekennzeichnet dadurch,
- daß er spezifisch einerseits gp300, gp140, gp125 und gp80 nach einem der Ansprüche 1 bis 4, die in durch ein Retrovirus des Typs HIV-2 infizierten Zellen vorliegen, und andererseits gp125, gp80 nach einem der Ansprüche 1 bis 4 und gp36, die in Extrakten eines Retrovirus des Typs HIV-2 vorliegen, erkennt und
- daß er die Proteine oder Glykoproteine von gesunden Zellen oder von durch ein Retrovirus des Typs HIV-1 infizierten Zellen nicht erkennt.

6. Monoklonaler Antikörper,
dadurch gekennzeichnet, daß er spezifisch mit dem Glykoprotein gp80 nach einem der Ansprüche 1 bis 4 reagiert, daß er nicht mit den Proteinen und Glykoproteinen von durch ein Retrovirus des Typs HIV-1 infizierten Zellen oder mit viralen Aggregaten von HIV-1 reagiert und daß er darüberhinaus die Glykoproteine gp300, gp140 und gp36 von HIV-2 erkennt.

7. Hybridom, das monoklonale Antikörper nach Anspruch 6 produziert.

8. Antigene Zusammensetzung für die spezifische in vitro-Diagnose einer Infektion aufgrund eines humanen Retrovirus des Typs HIV-2 in einer biologischen Probe, die nach dieser Infektion gebildete Antikörper enthalten könnte, dadurch gekennzeichnet, daß sie mindestens das Glykoprotein gp80 nach einem der Ansprüche 1 bis 4 enthält.

9. Immunogene Zusammensetzung,
dadurch gekennzeichnet, daß sie mindestens das Glykoprotein gp80 nach einem der Ansprüche 1 bis 4 in Verbindung mit einem pharmazeutisch annehmbaren Träger für die Herstellung von Impfstoffen enthält.

10. Verfahren zur in vitro-Diagnose einer Infektion, die spezifisch auf ein Retrovirus des Typs HIV-2 zurückgeführt werden kann, gekennzeichnet durch die Schritte:
- In-Kontakt-Bringen einer biologischen Probe, die nach einer Infektion durch ein Retrovirus des Typs HIV-2 gebildete Antikörper enthalten könnte, mit einem Glykoprotein gp80 nach einem der Ansprüche 1 bis 4 oder mit einer antigenen Zusammensetzung nach Anspruch 8 unter geeigneten Bedingungen, die die Bildung eines immunologischen Komplexes vom Antigen-Antikörper-Typ erlauben, und
- Nachweis des gegebenenfalls gebildeten Antigen-Antikörper-Komplexes.

11. Verfahren zur Herstellung eines antigenen Glykoproteins gp80 nach einem der Ansprüche 1 bis 4, gekennzeichnet durch die folgenden Schritte:
- Lyse von durch ein humanes Retrovirus HIV-2 infizierten Zellen und Trennung des Überstandes und der infizierten Zellen oder Lyse der durch Zentrifugation hergestellten, Viren enthaltenden Niederschläge,
- Inkubation des Zellextrakts und/oder des viralen Extrakts auf einer Immunaffinitätssäule, welche mit einem Immunadsorbens beschickt ist, das gereinigte Antikörper enthält, wie solche, die aus einem Serum eines durch das humane Retrovirus HIV-2 infizierten Kranken erhalten werden, wobei dieses Serum die Fähigkeit hat, stark mit den Hüllproteinen von HIV-2 zu reagieren, wobei diese Antikörper vorteilhafter an einen adaptierten Träger gekoppelt sind, in Anwesenheit eines Puffers und für eine ausreichend lange Zeitdauer, um die Bildung eines immunologischen Antigen-Antikörper-Komplexes zu erhalten,
- Waschen der Säule mit einem Puffer zum Entfernen der nicht auf dem Träger zurückgehaltenen Moleküle und
- Gewinnung der gewünschten antigenen Proteine.

12. Verfahren nach Anspruch 11 zur Herstellung des Glykoproteins nach einem der Ansprüche 1 bis 4,
dadurch gekennzeichnet, daß die Gewinnung der Glykoproteine bewerkstelligt wird durch Elektrophorese und durch Elektroelution.

13. Verfahren nach Anspruch 11 zur Herstellung des Glykoproteins nach einem der Ansprüche 1 bis 4,
dadurch gekennzeichnet, daß die Gewinnung der Glykoproteine erhalten wird durch
- Elution der an die vorstehend bezeichnete Immunaffinitätssäule gebundenen Proteine und
- Reinigung der so eluierten Produkte über eine Chromatographiesäule, die an den Trennungsträger gekoppelt monoklonale Antikörper, die gp80 erkennen, enthält.

14. Zusammenstellung oder Kit zum Nachweis von Antikörpern im Serum oder in einer anderen biologischen Probe eines Patienten, der durch ein humanes Retrovirus HIV-2 infiziert sein könnte, gekennzeichnet dadurch, daß sie bzw. er enthält:
- mindestens ein antigenes Protein und/oder Glykoprotein nach einem der Ansprüche 1 bis 4
- oder eine Zusammensetzung nach Anspruch 8
- oder weiters eine Mischung dieser unterschiedlichen Bestandteile und
- Mittel, die die Bildungsreaktion des immunologischen Komplexes zwischen den vorstehend genannten Antigenen und den gegebenenfalls in der zu untersuchenden biologischen Probe anwesenden Antikörpern ermöglichen, insbesondere, sofern erforderlich, einen oder mehrere Inkubationspuffer,
- eine negative Kontrollprobe und
- Mittel zum Nachweis des gebildeten Antigen-Antikörper-Komplexes.

15. Verfahren zum Nachweis einer Infektion durch das Retrovirus HIV-2 in einer biologischen Probe,
gekennzeichnet durch die Schritte:
- Behandlung der biologischen Probe auf eine Weise, daß die intrazellulären Proteine freigesetzt werden und
- Nachweis der Anwesenheit des Glykoproteins nach Anspruch 1 unter den freigesetzten intrazellulären Proteinen, beispielsweise durch Elektrophorese oder durch einen immunologischen Test mittels immunologisch hinsichtlich gp80 reaktiver Antikörper.
